# EUROPÄISCHE PATENTSCHRIFT

(11) **EP 2 086 944 B1**
(45) Veröffentlichungstag und Bekanntmachung des Hinweises auf die Patenterteilung: **13.01.2010**
(21) Anmeldenummer: 07822162.9
(22) Anmeldetag: 02.11.2007
(51) Int. Cl.: C07D 237/04, C07F 7/04

(54) **VERFAHREN ZUR HERSTELLUNG VON DIFLUORMETHYLPYRAZOLYLCARBOXYLATEN**
PROCESS FOR PREPARING DIFLUOROMETHYLPYRAZOLYL CARBOXYLATES
PROCÉDÉ DE FABRICATION DE DIFLUOROMÉTHYLPYRAZOLYLCARBOXYLATES

(30) Priorität: 03.11.2006 EP 06123461
(43) Veröffentlichungstag der Anmeldung: 12.08.2009
(73) Patentinhaber: BASF SE, 67056 Ludwigshafen (DE)
(72) Erfinder: RACK, Michael, 69214 Eppelheim (DE); SMIDT, Sebastian Peer, 68163 Mannheim (DE); LÖHR, Sandra, 67059 Ludwigshafen (DE); KEIL, Michael, 67251 Freinsheim (DE); DIETZ, Jochen, 68167 Mannheim (DE); RHEINHEIMER, Joachim, 67063 Ludwigshafen (DE); GROTE, Thomas, 67157 Wachenheim (DE); ZIERKE, Thomas, 67459 Böhl-iggelheim (DE); LOHMANN, Jan Klaas, 67063 Ludwigshafen (DE); SUKOPP, Martin, 68165 Mannheim (DE)
(86) Internationale Anmeldenummer: PCT/EP2007/061833
(87) Internationale Veröffentlichungsnummer: WO 2008/053043

(56) Entgegenhaltungen:
- WO-A-2005/123690
- US-A- 5 618 951

## Beschreibung

Die vorliegende Erfindung betrifft ein Verfahren zur Herstellung von Difluormethyl-substituierten Pyrazol-4-ylcarboxylaten.

Die WO 92/12970 beschreibt (3-Difluormethyl-1-methylpyrazol-4-yl)carboxamide und deren Verwendung als Fungizide. Die Herstellung erfolgt ausgehend von einem 2,2-Difluoracetessigester, der sukzessive mit Triethylorthoformiat und mit Methylhydrazin umgesetzt wird, wobei man den 3-Difluormethyl-1-methylpyrazol-4-carbonsäureesters erhält, welcher anschließend zur Carbonsäure verseift wird. Diese wird nach Umwandlung in das Säurechlorid mit einem geeigneten Amin zum entsprechenden Amid umgesetzt. Die Bereitstellung des als Edukt benötigten 4,4-difluorierten Acetessigesters ist jedoch vergleichsweise kostspielig und schwierig und stellt für dieses Verfahren ein Hindernis dar.

Die WO 2005/044804 beschreibt Alkylester fluormethylsubstituierter heterocyclischer Carbonsäuren sowie deren Herstellung durch Halogenaustausch an entsprechenden chlormethylsubstituierten heterocyclischen Carbonsäureestem und deren Weiterverarbeitung zu Aniliden der fluormethylsubstituierten heterocyclischen Carbonsäuren. Die Verwendung von Fluorierungsmitteln ist jedoch kostspielig und stellt spezielle Anforderungen an Sicherheitsmaßnahmen und die verwendeten Apparaturen.

Der vorliegenden Erfindung liegt somit die Aufgabe zugrunde, alternative Verfahren zur Herstellung von (3-Difluormethylpyrazol-4-yl)carboxylaten und deren Derivaten bereitzustellen, die von Produkten ausgehen, deren Bereitstellung weniger aufwändig ist als beispielsweise die Bereitstellung von 4,4-Difluoracetessigestern.

Überraschenderweise wurde gefunden, dass diese Aufgabe durch Umsetzung von 4,4,4-Trihalogen-substituierten Acetessigesterderivaten der im Folgenden definierten Formel II mit Chlorsilanen in Gegenwart von Magnesium oder anderen Metallen der 1, 2, 3, 4 oder 12 Gruppe des Periodensystems und anschließende Umsetzung des Reaktionsprodukts mit einem Hydrazin oder Hydrazinderivat gelöst wird.

Gegenstand der vorliegenden Erfindung ist somit ein Verfahren zur Herstellung von Difluormethyl-substituierten Pyrazol-4-ylcarboxylaten der allgemeinen Formel I, 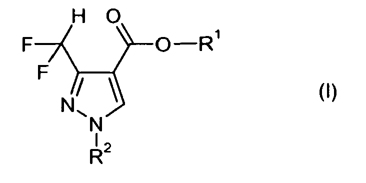 worin
- R¹: für C₁-C₈-Alkyl, C₃-C₈-Cycloalkyl, C₁-C₄-Alkoxy-C₁-C₄-alkyl, C₃-C₈-Cycloalkoxy-C₁-C₄-alkyl, C₂-C₈-Alkenyl oder für gegebenenfalls durch 1, 2 oder 3 unabhängig voneinander unter C₁-C₄-Alkyl, C₁-C₄-Alkoxy oder Nitro ausgewählten Substituenten R^{y1} substituiertes Benzyl steht; und
- R²: für Wasserstoff, C₁-C₄-Alkyl, Benzyl oder Phenyl steht, wobei die beiden letztgenannten Substituenten unsubstituiert oder gegebenenfalls durch 1, 2 oder 3 unabhängig voneinander unter Halogen, CN, Nitro, C₁-C₄-Alkyl, C₁-C₄-Haloalkyl, C₁-C₄-Alkoxy oder C₁-C₄-Haloalkoxy ausgewählte Substituenten R^{y2} substituiert sein können;
wobei man
a) eine Verbindung der allgemeinen Formel II 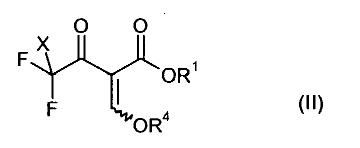 worin X für Fluor, Chlor oder Brom steht, R¹ eine der zuvor gegebenen Bedeutungen besitzt und R⁴ für C₁-C₈-Alkyl, C₃-C₈-Cycloalkyl, C₂-C₈-Alkenyl, Benzyl oder Phenyl steht, mit einer Silanverbindung der allgemeinen Formel R³ₙSiCl₍₄₋ₙ₎, worin n für 1, 2 oder 3 steht und die Substituenten R³ unabhängig voneinander ausgewählt sind unter C₁-C₈-Alkyl oder Phenyl, und mit einem Metall umsetzt, das unter den Metallen der 1, 2, 3, 4 und 12 Gruppe des Periodensystems ausgewählt ist und ein Redoxpotential von weniger als -0,7 V, bezogen auf eine Normalwasserstoffelektrode (bei 25 °C und 101,325 kPa), aufweist; und
b) das Reaktionsgemisch aus Schritt a) mit einer Verbindung der allgemeinen Formel III

   R²HN-NH₂ (III)

   umsetzt, worin R² eine der zuvor gegebenen Bedeutungen besitzt.

Das erfindungsgemäße Verfahren ist mit einer Reihe von Vorteilen verbunden. Es liefert die Pyrazolverbindung der Formel II mit hoher Ausbeute und, im Falle der Umsetzung mit substituierten Hydrazinen III (R² ≠ H), in hoher Regioselektivität. Zudem kann auf teure Ausgangsmaterialien, Difluormethyl-Carbonylverbindungen wie 2,2-Difluoracetessigester verzichtet werden und anstelle dessen die sehr viel preiswerteren Trifluormethylcarbonylverbindungen und Halodifluormethylverbindungen wie 2,2,2-Trifluoracetessigester oder 2-Chlor-2,2-difluoracetessigester eingesetzt werden.

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen. Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom.

Beispiele anderer Bedeutungen sind:
Der Begriff "C₁-C₆-Alkyl", wie hierin und in den Begriffen C₁-C₆-Alkoxy, C₁-C₆-Alkylamino, Di(C₁-C₆-alkyl)amino, C₁-C₆-Alkylthio, C₁-C₆-Alkylsulfonyl, C₁-C₆-Alkylsulfoxyl, C₁-C₆-Alkylcarbonyl, C₁-C₆-Alkoxycarbonyl, und C₁-C₆-Alkylcarbonyloxy verwendet, bezeichnet eine gesättigte, geradkettige oder verzweigte Kohlenwasserstoffgruppe, umfassend 1 bis 6 Kohlenstoffatome, speziell 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.
Der Begriff "C₁-C₆-Haloalkyl", wie hierin und in den Haloalkyleinheiten von C₁-C₆-Haloalkoxy verwendet, beschreibt geradkettige oder verzweigte Alkylgruppen mit 1 bis 6 Kohlenstoffatomen, wobei die Wasserstoffatome dieser Gruppen teilweise oder vollständig durch Halogenatome ersetzt sind, beispielsweise C₁-C₄-Haloalkyl, wie Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl etc.
Der Begriff "C₁-C₆-Alkoxy" beschreibt geradkettige oder verzweigte gesättigte Alkylgruppen, umfassend 1 bis 6 Kohlenstoffatome, die über ein Sauerstoffatom gebunden sind. Beispiele umfassen C₁-C₆-Alkoxy wie beispielsweise Methoxy, Ethoxy, OCH₂-C₂H₅, OCH(CH₃)₂, n-Butoxy, OCH(CH₃)-C₂H₅, OCH₂-CH(CH₃)₂, OC(CH₃)₃, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, etc.
Der Begriff "C₁-C₄-Alkoxy-C₁-C₄-alkyl", wie hierin verwendet, beschreibt C₁-C₄-AlkylRadikale, wobei ein Kohlenstoffatom an ein C₁-C₄-Alkoxy-Radikal gebunden ist. Beispiele hierfür sind CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)-propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)-butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl, 4-(1,1-Dimethylethoxy)butyl, etc.
Der Begriff "C₁-C₆-Alkylcarbonyl", wie hierin verwendet, beschreibt eine geradkettige oder verzweigte gesättigte Alkylgruppe, umfassend 1 bis 6 Kohlenstoffatome, die terminal oder intern über das Kohlenstoffatom einer Carbonyleinheit gebunden ist.
Der Begriff "C₁-C₆-Alkoxycarbonyl", wie hierin verwendet, beschreibt eine geradkettige oder verzweigte Alkoxygruppe, umfassend 1 bis 6 Kohlenstoffatome, die über das Kohlenstoffatom einer Carbonyleinheit gebunden ist.
Der Begriff "C₁-C₆-Alkylcarbonyloxy", wie hierin verwendet, beschreibt geradkettige oder verzweigte gesättigte Alkylgruppen, umfassend 1 bis 6 Kohlenstoffatome, die terminal oder intern über das Kohlenstoffatom der Carbonyloxyeinheit gebunden ist.
Der Begriff "C₂-C₆-Alkenyl", wie hierin und für die Alkenyleinheiten von C₂C₆-Alkenyloxy verwendet, beschreibt geradkettige und verzweigte ungesättigte Kohlenwasserstoffradikale, umfassend 2 bis 6 Kohlenstoff Atome und wenigstens eine Kohlenstoff-Kohlenstoff-Doppelbindung, wie beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.
Der Begriff "C₃-C₁₄-Cycloalkyl" wie hierin verwendet, beschreibt mono- , bi- oder polycyclische Kohlenwasserstoffradikale, umfassend 3 bis 8 Kohlenstoffatome, speziell 3 bis 6 Kohlenstoffatome. Beispiele monocyclischer Radikale umfassen Cyclopropyl, Cyclobutyl, Cyclopentyl, Cyclohexyl, Cycloheptyl oder Cyclooctyl. Beispiele bicyclischer Radikale umfassen Bicyclo[2.2.1]heptyl, Bicyclo[3.1.1]heptyl, Bicyclo[2.2.2]octyl und bicyclo[3.2.1]octyl. Beispiele tricyclischer Radikale sind Adamantyl und Homoadamantyl.

Die Doppelbindung in Verbindung II und ebenso in den nachfolgend definierten Formeln A und B kann E- oder Z-Konfiguration aufweisen (bzw. cis- oder trans-Konfiguration, bezogen auf die relative Anordnung der Gruppe OR⁴ und des Trifluoracetyl-Rests).

In einer bevorzugten Ausführungsform des erfindungsgemäßen Verfahrens steht R¹ in den Formeln I und II für C₁-C₄-Alkyl oder Benzyl, insbesondere für Methyl, Ethyl oder Isopropyl; speziell steht R¹ für Ethyl.

Weiterhin ist es für das erfindungsgemäßen Verfahren von Vorteil, wenn R⁴ in Formel II ausgewählt ist unter C₁-C₄-Alkyl oder Benzyl und insbesondere unter Methyl, Ethyl, Isopropyl oder Benzyl; speziell steht R⁴ für Ethyl steht.

In Formel II steht X insbesondere für Fluor oder Chlor. In einer besonders bevorzugten Ausführungsform der Erfindung steht X für Fluor.

R² steht vorzugsweise für Wasserstoff oder C₁-C₄-Alkyl; speziell steht R² für Methyl. Demzufolge ist die Verbindung der allgemeinen Formel III bevorzugt ausgewählt unter C₁-C₄-Alkylhydrazin oder Hydrazin; speziell handelt es sich bei der Verbindung der allgemeinen Formel III um Methylhydrazin oder um Hydrazinhydrat.

Alle hier beschriebenen Umsetzungen werden in für derartige Reaktionen üblichen Reaktionsgefäßen durchgeführt, wobei die Reaktionsführung sowohl kontinuierlich als auch diskontinuierlich ausgestaltet werden kann. In der Regel wird man die jeweiligen Reaktionen drucklos durchführen. Bei niedrig siedenden Lösungsmitteln kann die jeweilige Reaktion auch unter Druck durchgeführt werden.

### Schritt a)

Zur Durchführung von Schritt a) des erfindungsgemäßen Verfahrens wird die Verbindung der Formel II mit der Silanverbindung und mit einem Metall der 1, 2, 3, 4 und 12 Gruppe des Periodensystems, das ein Redoxpotential von weniger als -0,7 V, bezogen auf eine Normalwasserstoffelektrode (bei 25 °C und 101,325 kPa), aufweist, vorzugsweise mit einem Metall der 1., 2. oder 3. Hauptgruppe des Periodensystems oder Zink, oder speziell mit Magnesium umgesetzt.

Ohne an eine Theorie gebunden zu sein nimmt man an, dass hierbei primär ein silyliertes Enol der Formel A erhalten wird, das möglicherweise, abhängig von den gewählten Reaktionsbedingungen oder bei der Aufarbeitung der Reaktion oder im Verlauf der weiteren Umsetzung in Schritt b) intermediär zur Verbindung B hydrolysiert.

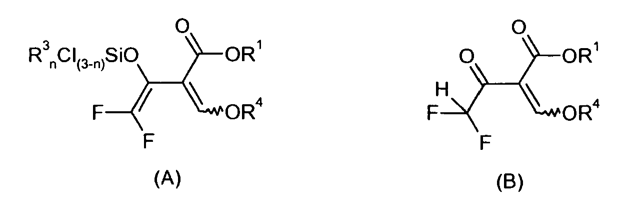

Die Verbindungen A sind in der Reaktionslösung nachweisbar und können in einigen fällen isoliert werden. Die Verbindungen A und ihre Lösungen sind daher ebenfalls Gegenstand der vorliegenden Erfindung. Bezüglich bevorzugter Bedeutungen von R¹, R³ und R⁴ in den Verbindungen A gilt das zuvor und im Folgenden gesagte in analoger Weise.

Die Umsetzung kann grundsätzlich in Analogie zu der in Organic Letters, 2001, 3(20), 3103-3105 beschriebenen Reaktion durchgeführt werden, welche die Herstellung von 1-Ethoxy-3-trimethylsilyloxy-4,4-difluorbutadien beschreibt. Wenn X für Chlor oder Fluor steht, kann die Umsetzung insbesondere in Analogie zu den in Tetrahedron Letters, 1983, Vol. 24, No.5, 507-510. J. Chem. Soc. Perkin Trans. I, 1988, 1149-1153, J. Org. Chem. 1995, 60, 5570-5578, J. Org. Chem. 2006, 71, No. 15, 5468-5473 und US 5,618,951 beschriebenen Methoden durchgeführt werden.

Beispiele für Metalle der 1, 2, 3, 4 und 12 Gruppe des Periodensystems, die ein Redoxpotential von weniger als -0,7 V, z. B. < -0,7 bis -3,0 V, bezogen auf eine Normalwasserstoffelektrode (bei 25 °C und 101,325 kPa), aufweisen sind Alkalimetalle, insbesondere Lithium, Natrium oder Kalium, Erdalkalimetalle, insbesondere Magnesium oder Kalzium, weiterhin Aluminium, Titan, Zirkon und Zink. Bevorzugte Metalle sind Natrium, Magnesium und Zink, wobei Magnesium besonders bevorzugt ist, insbesondere wenn X für Fluor steht. Gleichermaßen besonders bevorzugt ist Zink, insbesondere wenn X für Chlor oder Brom steht.

Es hat sich als vorteilhaft erwiesen, wenn man in Schritt a) eine Silanverbindung R³ₙSiCl₍₄₋ₙ₎ einsetzt, worin n für 2 oder 3 steht. Besonders bevorzugt stehen die Substituenten R³ in diesen Silanverbindungen unabhängig voneinander für C₁-C₄-Alkyl, insbesondere Methyl, Ethyl, Isopropyl, tert.-Butyl. Insbesondere handelt es sich um eine Silanverbindung, worin n für 3 steht. Hierin können die 3 Reste R³ gleich oder verschieden sein, wobei solche Silanverbindungen bevorzugt sind, worin 2 der Reste R³ für Methyl stehen und der verbleibende Rest R³ für C₁-C₄-Alkyl steht. In diesem Fall handelt es sich bei der Silanverbindung vorzugsweise um Trimethylsilylchlorid, Ethyldimethylsilylchlorid, Dimethylpropylsilylchlorid, Dimethylisopropylsilylchlorid, n-Butyldimethylsilylchlorid, 2-Butyldimethylsilylchlorid, (2-Methylpropyl)dimethylsilylchlorid, oder tert.-Butyldimethylsilylchlorid und ganz besonders bevorzugt Trimethylsilylchlorid. Beispiele für bevorzugte Silanverbindungen, worin n für 2 steht, sind Dimethyldichlorsilan und Diethyldichlorsilan. In einer ganz besonders bevorzugten Ausführungsform handelt es sich bei der Silanverbindung R³ₙSiCl₍₄₋ₙ₎ um Trimethylsilylchlorid.

Vorzugsweise setzt man die Silanverbindung in wenigstens äquimolaren Mengen oder im Überschuss bezogen auf die Verbindung II ein, wobei die Menge an Silanverbindung in der Regel 5 Mol, insbesondere 3,5 Mol, pro mol Verbindung II, nicht überschreiten wird. Vorzugsweise setzt man 1,1 bis 3,5 Mol, insbesondere etwa 1,2 bis 2,5 Mol der Verbindungen der Silanverbindung je Mol der Verbindung II ein.

In der Regel setzt man die Silanverbindung in einer Menge von wenigstens 0,8 Mol, pro Mol Metall ein. Vorzugsweise setzt man die Silanverbindung, in wenigstens äquimolaren Mengen oder im Überschuss, bezogen auf das Metall ein, wobei ein größerer Überschuss, z. B. mehr als 200 Mol-%, bezogen auf die Menge an Metall in der Regel nicht erforderlich ist. Vorzugsweise setzt man 0,8 bis 3 Mol, insbesondere etwa 0,9 bis 3 Mol und speziell 1 bis 2 Mol der Silanverbindung je Mol Metall ein.

Bezogen auf die Verbindung II setzt man das Metall in der Regel in wenigstens äquimolarer Menge ein, z. B. in einer Menge von 1 bis 5 Mol, häufig 1,1 bis 4 Mol und speziell 1,5 bis 3 Mol, pro mol Verbindung II.

Vorzugsweise führt man Schritt a) in weitgehender Abwesenheit von Wasser, d. h. einem trockenen organischen Lösungsmittel durch. Hier und im Folgenden bedeutet trocken, dass das Lösungsmittel einen Wassergehalt von weniger als 500 ppm und insbesondere nicht mehr als 100 ppm aufweist. Beispiele für geeignete organische Lösungsmittel sind aprotische polare Lösungsmittel, z. B. cyclische oder acyclische Ether wie Diethylether, tert.-Butylmethylether (MTBE), Diisopropylether, Tetrahydrofuran (THF) oder Dioxan, cyclische oder acyclische Amide, z. B. N-C₁-C₄-Alkyllactame wie N-Methylpyrrolidon oder N-Di(C₁-C₄-alkyl)amide aliphatischen C₁-C₄-Carbonsäuren wie Dimethylformamid oder Dimethylacetamid oder, aprotischen Harnstoffderivaten, d. h. N,N,N',N'-Tetraalkylharnstoffen oder N,N'-dialkylierten cyclischen Harnstoffen, wie N,N,N',N'-Tetra(C₁-C₄-alkyl)alkylharnstoffen, 1,3-Di(C₁-C₄-alkyl)hexahydropyrimidin-2-on oder 1,3-Di(C₁-C₄-alkyl)imidazolin-2-on, z.B.Tetramethytharnstoff, 1,3-Dimethylhexahydropyrimidin-2-on (Dimethylpropylenharnstoff) oder 1,3-Dimethylimidazolin-2-on (DMI), sowie Mischungen der zuvor genannten Lösungsmittel. Geeignet sind auch Mischungen der vorgenannten aprotisch polaren organischen Lösungsmittel mit unpolaren, aprotischen Lösungsmitteln, z. B. mit aromatischen oder (cyclo)aliphatischen Kohlenwasserstoffen wie Toluol, Xylole, Hexan, Cyclohexan und dergleichen, wobei in diesen Mischungen vorzugsweise das aprotisch polare Lösungsmittel vorzugsweise wenigstens 50 Vol.-%, insbesondere wenigstens 70 Vol.-% der Gesamtlösungsmittelmenge ausmacht. Vorzugsweise erfolgt die Umsetzung in einem aprotisch polaren Lösungsmittel, das unter cyclischen oder acyclischen Amiden, insbesondere N-C₁-C₄-Alkyllactamen wie N-Methylpyrroldion, N-Di(C₁-C₄-alkyl)amiden aliphatischer C₁-C₄-Carbonsäure wie Dimethylformamid oder Dimethylacetamid, und aprotischen Harnstoffderivaten wie N,N,N',N'-Tetra(C₁-C₄-alkyl)alkylharnstoffen, 1,3-Di(C₁-C₄-alkyl)hexahydropyrimidin-2-on oder 1,3-Di(C₁-C₄-alkyl)imidazolin-2-on, z. B. Tetramethylharnstoff, 1,3-Dimethylhexahydropyrimidin-2-on (Dimethylpropylenharnstoff) oder 1,3-Dimethylimidazolin-2-on (DMI) und Mischungen dieser Lösungsmittel ausgewählt ist. Besonders bevorzugte Lösungsmittel sind die aprotischen Harnstoffverbindungen und speziell DMI.

Vorzugsweise führt man die Umsetzung in Schritt a) bei Temperaturen von -10 bis +60 °C durch. Vorzugsweise wird man dafür Sorge tragen, dass eine Reaktionstemperatur von 50 °C, insbesondere 30 °C nicht überschritten wird.

Die Umsetzung erfolgt in an sich üblicher Weise, indem man die Reagenzien, d. h. die Verbindung II, die Silanverbindung und das Metall, vorzugsweise in einem geeigneten Lösungsmittel in einem Reaktionsgefäß miteinander in Kontakt bringt, wobei man in der Regel das Metall im Reaktionsgefäß und gegebenenfalls die Silanverbindung vorlegt. Von Vorteil ist es, wenn wenigstens ein Teil der Silanverbindung, z. B. wenigstens 20 %, insbesondere wenigstens 50 % und das Metall in einem für die Umsetzung geeigneten, vorzugsweise trockenen organischen Lösungsmittel im Reaktionsgefäß befindlich sind, bevor man die Verbindung II zugibt.

In einer bevorzugten Vorgehensweise legt man das Metall und die Silanverbindung in einem für die Umsetzung geeigneten, vorzugsweise trockenen organischen Lösungsmittel vor. Das Metall wird hierbei typischerweise in partikulärer Form, beispielsweise in Form von Spänen, Pulvern oder Granalien eingesetzt. Die Reihenfolge, in der die Bestandteile vorgelegt werden, ist dabei von untergeordneter Bedeutung. Gegebenenfalls führt man eine Aktivierung des Metalls durch. Anschließend gibt man, gegebenenfalls unter Kühlung zur Abführung der Reaktionswärme, die Verbindung der Formel II in Reinform oder als Lösung zu, wobei die Zugaberate vorzugsweise so gewählt wird, dass die oben angegebenen Temperaturen möglichst eingehalten und insbesondere nicht überschritten werden. Verwendet man eine Lösung der Verbindung der Formel II, liegt die Konzentration an Verbindung II typischerweise im Bereich von 10 bis 90 Gew.-%, bezogen auf das Gesamtgewicht der Lösung. Bei dem für die Lösung verwendeten Lösungsmittel handelt es sich typischerweise um das für die Reaktion verwendeten Lösungsmittel.

In einer anderen, ebenfalls bevorzugten Vorgehensweise legt man das Metall in einem für die Umsetzung geeigneten, vorzugsweise trockenen organischen Lösungsmittel vor, führt gegebenenfalls eine Aktivierung durch und gibt dann sukzessive zunächst die Silanverbindung und dann die Verbindung II in das Reaktionsgefäß, wobei die Silanverbindung und die Verbindung II in Reinform oder als Lösung in dem für die Reaktion gewünschten Lösungsmittel zugegeben werden können.

In einer weiteren, ebenfalls bevorzugten Vorgehensweise legt man das Metall in einem für die Umsetzung geeigneten, vorzugsweise trockenen organischen Lösungsmittel vor, führt gegebenenfalls eine Aktivierung durch und gibt dann gegebenenfalls einen Teil der Silanverbindung, z. B. 1 bis 30 % zu, und gibt dann die Silanverbindung bzw. die Restmenge der Silanverbindung und die Verbindung II, zusammen oder über getrennte Zuläufe, in das Reaktionsgefäß, wobei die Silanverbindung und die Verbindung II in Reinform oder als Lösung in dem für die Reaktion gewünschten Lösungsmittel zugegeben werden können.

Alternativ kann man auch die Verbindung II und das Metall in einem für die Reaktion geeigneten Lösungsmittel vorlegen und hierzu die Silanverbindung in Reinform oder als Lösung in dem für die Reaktion geeigneten Lösungsmittel zugeben.

Je nach Reaktivität der Verbindungen II, der Silanverbindung, dem Metall und der Reaktionstemperatur liegt Zeit für die Zugabe der Verbindung II bzw. der Silanverbindung in der Regel im Bereich von 5 bis 240 Minuten, häufig im Bereich von 10 bis 120 Minuten, bevorzugt im Bereich von 20 bis 60 Minuten. Gegebenenfalls kann sich hiernach eine Nachreaktionsphase anschließen, die in der Regel im Bereich von 10 Minuten bis 360 Minuten, häufig im Bereich von 15 Minuten bis 240 Minuten und insbesondere im Bereich von 20 Minuten bis 180 Minuten. Die für eine vollständige Umsetzung erforderliche gesamte Reaktionszeit (Zugabedauer + etwaige Nachreaktionsphase) liegt in der Regel im Bereich von 20 Minuten bis 10 Stunden, häufig im Bereich von 30 Minuten bis 6 h und vorzugsweise im Bereich von 1 bis 5 Stunden. Die Umsetzung der Verbindung II ist in der Regel quantitativ oder nahezu quantitativ (> 95 % Umsatz).

Es hat sich bewährt, das im Reaktionsgefäß vorgelegte Metall vor der Zugabe der Verbindung II bzw. der Silanverbindung zu aktivieren, z. B. durch Ultraschallbehandlung oder auf chemischem Wege, z. B. durch Behandlung mit Brom, Iod, Trichlormethan oder Dibrommethan.

Für die weitere Umsetzung kann eine Aufarbeitung der Reaktionsmischung und eine Isolierung des Reaktionsprodukts aus Schritt a) durchgeführt werden. Zur quantitativen Isolierung von Verbindungen der Formel A wird man in der Regel unter schwach basischen oder wasserfreien Bedingungen Arbeiten, um eine vorzeitige Hydrolyse zu vermeiden. Sofern man unter Zusatz von Wasser aufarbeitet, findet zumindest teilweise eine Hydrolyse zur Verbindung B statt und man erhält die Verbindung B, gegebenenfalls im Gemisch mit der Verbindung A. Vorzugsweise wird eine vorzeitige Hydrolyse der Verbindung A vermieden.

Eine Isolierung der gebildeten Verbindungen ist für die Umsetzung in Schritt b) nicht erforderlich. Vielmehr hat es sich als günstig erwiesen, auf eine Isolierung der Reaktionsprodukte zu verzichten. Daher erfolgt in einer bevorzugten Ausführungsform des erfindungsgemäßen Verfahrens die weitere Umsetzung in Schritt b) ohne vorherige Isolierung der Reaktionsprodukts A. Vorzugsweise wird man vor der weiteren Umsetzung überschüssige Silanverbindung teilweise oder insbesondere weitgehend, d. h. zumindest bis zu einer Restmenge von weniger als 10 Mol-%, bezogen auf die eingesetzte Verbindung II, oder vollständig entfernen, z. B. auf destillativem Wege. Gegebenenfalls wird man auch weitere volatile Bestandteile der Reaktionsmischung, z. B. das Lösungsmittel teilweise oder insbesondere vollständig entfernen.

Die in Schritt a) eingesetzten Verbindungen der allgemeinen Formel II sind kommerziell erhältlich oder können beispielsweise in Analogie zu der für (2-Ethoxymethylen-4,4,4-trifluor)acetessigester in Journal of Medicinal Chemistry, 2000, Vol. 43, No. 21 beschriebenen Reaktion aus einem Tris(halogen)acetoessigsäureester VII und einem geeigneten Orthoformiat VIII, in der Regel ein Tri-(C₁-C₄-alkyl)orthoformiat bereitgestellt werden. Die Reaktion ist in dem folgenden Schema dargestellt:

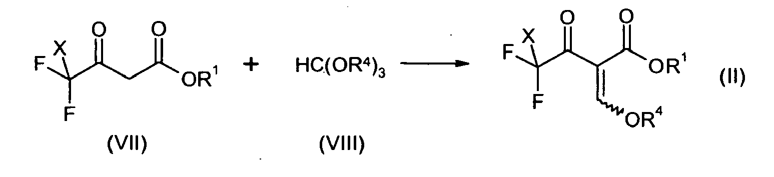

In diesem Schema haben X, R¹ und R⁴ die zuvor genannten Bedeutungen. Ein hierfür geeignetes Orthoformiat ist insbesondere Triethylorthoformiat (R⁴ = Ethyl). In der Regel führt man die Umsetzung von VII mit VIII in einer Weise durch, dass das bei der Umsetzung gebildete C₁-C₄-Alkanol aus dem Reaktionsgleichgewicht entfernt wird, beispielsweise indem man es abdestilliert oder chemisch bindet, beispielsweise indem man die Umsetzung in Gegenwart eines Anhydrids einer Carbonsäure, z. B. einer C₂-C₄-Alkancarbonsäure wie Essigsäureanhydrid durchführt.

In der Regel wird zur Umsetzung die Verbindung VIII im Überschuss, bezogen auf die Stöchiometrie der Reaktion eingesetzt. Insbesondere setzt man 1, 1 bis 5 Mol und speziell 1,2 bis 2 Mol Orthoformiat VIII, bezogen auf 1 Mol Verbindung VII ein.

Die Umsetzung von VII mit VIII erfolgt üblicherweise bei erhöhter Temperatur, häufig im Bereich von 80 bis 180 °C, insbesondere im Bereich von 100 bis 150 °C. Gegebenenfalls kann man eine Säure, z. B. eine organische Sulfonsäure wie p-Toluolsulfonsäure, als Katalysator zusetzen. In einer bevorzugten Ausgestaltung erfolgt die Umsetzung von VII mit dem Orthoformiat VIII in Acetanhydrid.

In der Regel wird man das die Verbindung II vor ihrem Einsatz im erfindungsgemäßen Verfahren reinigen, insbesondere von nicht umgesetzten Ausgangsmaterialien VII und/oder VIII abtrennen. Dies kann in einfacher Weise, z. B. durch fraktionierte Destillation erfolgen.

### Schritt b)

Die Herstellung von Difluormethyl-substituierten Pyrazol-4-ylcarboxylaten der allgemeinen Formel I in Schritt b) des erfindungsgemäßen Verfahrens erfolgt durch Umsetzung des Reaktionsprodukts aus Schritt a), gegebenenfalls nach einer Isolierung oder Aufreinigung der dabei erhaltenen Verbindungen, oder insbesondere durch Umsetzung der in Schritt a) erhaltenen Reaktionslösung, vorzugsweise nach Abtrennung überschüssiger Silanverbindung, mit einer Hydrazinverbindung der Formel IV.

Die Umsetzung in Schritt b) kann grundsätzlich in Analogie zu der WO 92/12970 beschriebenen Umsetzung von 3-(Difluormethyl-1-methylpyrazol-4-yl)ethylcarboxylat mit Methylhydrazin erfolgen.

Vorzugsweise setzt man Hydrazin oder ein Hydrazinderivat der allgemeinen Formel III, in wenigstens äquimolaren Mengen oder im Überschuss ein, wobei ein größerer Überschuss an Verbindung III, z. B. mehr als 20 Mol-%, bezogen auf ein Mol der in Schritt a) eingesetzten Verbindung II, in der Regel nicht erforderlich ist. Vorzugsweise setzt man 1,0 bis 1,2 Mol, insbesondere etwa 1,01 bis 1,1 Mol der Hydrazinverbindung III je Mol Verbindung II ein.

Bevorzugt handelt es sich bei der Hydrazinverbindung der Formel III um ein C₁-C₄-Alkylhydrazin oder Hydrazin bzw. Hydrazinhydrat; speziell handelt es sich bei der Verbindung der allgemeinen Formel III um Methylhydrazin oder um Hydrazinhydrat.

Zur Umsetzung des Reaktionsprodukts aus Schritt a) mit der Hydrazinverbindung III wird man in der Regel so vorgehen, dass man das Reaktionsprodukt aus Schritt a) vermischt, vorzugsweise indem man das Reaktionsprodukt, gegebenenfalls in Form einer Lösung in einem organischen Lösungsmittel, z. B. in Form der Reaktionslösung, gegebenenfalls nach Abtrennung überschüssiger Silanverbindung, zu der Hydrazinverbindung der Formel III gibt. Vorzugsweise legt man die Hydrazinverbindung der Formel III als Lösung in einem organischen Lösungsmittel oder Lösungsmittel/Wasser-Gemisch vor. Alternativ kann man auch so vorgehen, dass man die Hydrazinverbindung der Formel III, vorzugsweise als Lösung in einem organischen Lösungsmittel oder Lösungsmittel/Wasser-Gemisch, zu dem Reaktionsprodukt aus Schritt a) oder einer Lösung davon in einem organischen Lösungsmittel oder Lösungsmittel/Wasser-Gemisch gibt.

Für die Umsetzung in Schritt b) geeignete organische Lösungsmittel sind beispielsweise:
- protisch polare Lösungsmittel, z. B. aliphatische Alkohole mit vorzugsweise 1 bis 4 Kohlenstoffatomen, wie Methanol, Ethanol, n-Propanol, Isopropanol, n-Butanol, Isobutanol oder tert.-Butanol,
- aromatische Kohlenwasserstoffe, wie Benzol, Toluol, Xylole, Cumol, Chlorbenzol, Nitrobenzol oder tert.-Butylbenzol,
- aprotische polare Lösungsmittel, z. B. cyclische oder acyclische Ether wie Diethylether, tert.-Butylmethylether (MTBE), Tetrahydrofuran (THF) oder Dioxan, cyclische oder acyclische Amide, wie Dimethylformamid, Dimethylacetamid, N-Methylpyrrolidon oder Tetramethylharnstoff, oder aliphatische Nitrile wie Acetonitril oder Propionitril,
- sowie Mischungen der zuvor genannten Lösungsmittel.

Vorzugsweise erfolgt die Umsetzung in einem protisch polaren Lösungsmittel, insbesondere in einem C₁-C₄-Alkohol und besonders bevorzugt in Methanol, Ethanol, oder in Acetonitril, oder in einer Mischung eines protisch polaren Lösungsmittels mit einem aprotisch polaren Lösungsmittel oder in einem Gemisch dieser Lösungsmittel mit Wasser.

Vorzugsweise erfolgt die Umsetzung in Schritt b) in Gegenwart von Wasser. Hierbei sind bereits geringe Wassermengen von 0,1 Vol.-%, bezogen auf dies Gesamtlösungsmittelmenge (organisches Lösungsmittel + Wasser), ausreichend. In der Regel wird die Wassermenge 50 Vol.-%, häufig 30 Vol.-%, insbesondere 15 Vol.-%, bezogen auf die Gesamtmenge an organischem Lösungsmittel + Wasser nicht überschreiten und liegt häufig im Bereich von 0,1 bis 50 Vol.-%, vorzugsweise im Bereich von 0,5 bis 30 Vol.-%, insbesondere im Bereich von 1 bis 15 Vol.-% , bezogen auf die Gesamtmenge an organischem Lösungsmittel + Wasser. In einer besonders bevorzugten Ausführungsform der Erfindung führt man die Umsetzung in Schritt b) in einem Gemisch aus C₁-C₄-Alkanol und Wasser und speziell in einem Methanol-Wasser-Gemisch durch. Bezüglich der Volumenverhältnisse von Alkanol zu Wasser gilt das zuvor Gesagte.

Vorzugsweise führt man die Umsetzung bei Temperaturen von -80 bis +100 °C durch. In der Regel ist die obere Temperaturgrenze der Siedepunkt des jeweiligen Lösungsmittels bei druckloser Reaktionsführung. Vorzugsweise wird man eine Reaktionstemperatur von 60 °C und insbesondere 40 °C nicht überschreiten. Häufig wird man die Reaktion aus praktischen Gründen bei Raumtemperatur durchführen. In einer speziellen Ausführungsform wird vor Beginn der Umsetzung zunächst eine Temperatur von -60 bis 0 °C, insbesondere -60 bis -20 °C eingestellt und im Verlauf der Reaktion auf eine Temperatur von 0 bis 60 °C, insbesondere 10 bis 40 °C erwärmt.

Abhängig von der Reaktionstemperatur liegt die für eine vollständige Umsetzung erforderliche Reaktionszeit typischerweise im Bereich von 1 bis 48 Stunden und vorzugsweise im Bereich von 4 bis 24 Stunden.

Die Aufarbeitung der Reaktionsmischung und Gewinnung der Pyrazolverbindung der allgemeinen Formel I erfolgt in üblicher Weise, beispielsweise durch Entfernen des Lösungsmittels, z. B. durch Destillation oder durch eine wässrig extraktive Aufarbeitung oder durch eine Kombination dieser Maßnahmen. Eine weitere Reinigung kann beispielsweise durch Kristallisation oder durch Chromatographie erfolgen. Häufig fällt das Produkt jedoch bereits in einer Reinheit an, die weitere Reinigungsverfahren überflüssig macht.

Schritt b) des erfindungsgemäßen Verfahrens liefert die Verbindungen der allgemeinen Formel I in guten bis sehr guten Ausbeuten von in der Regel wenigstens 70 %.

Ein weiterer Gegenstand der vorliegenden Erfindung betrifft ein Verfahren zur Herstellung einer Verbindung der allgemeinen Formel IV 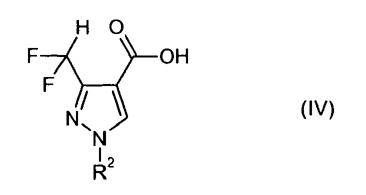 worin R² eine der zuvor gegebenen Bedeutungen besitzt. Das Verfahren zur Herstellung der Verbindung IV umfasst
i) die Bereitstellung einer Verbindung der Formel I nach dem erfindungsgemäßen Verfahren wie beschrieben und
ii) eine Hydrolyse der Verbindung I.

Die Hydrolyse kann unter Säurekatalyse oder basisch oder in sonstiger Weise durchgeführt werden. Die Verbindung I kann als solche, d. h. nach Isolierung eingesetzt werden. Es ist jedoch auch möglich das in Schritt b) erhaltene Reaktionsgemisch, gegebenenfalls nach Abtrennung flüchtiger Bestandteile wie Lösungsmittel, ohne weitere Reinigung zur Hydrolyse einzusetzen.

Zur basischen Hydrolyse der Verbindung I wird man typischerweise die Verbindung der Formel I mit einem Alkalimetallhydroxid wie Natriumhydroxid, Kaliumhydroxid oder Lithiumhydroxid, bevorzugt mit einer wässrigen Alkalimetallhydroxid-Lösung, speziell einer wässrigen NaOH-Lösung oder einer wässrigen KOH-Lösung, bis zur vollständigen Hydrolyse des Esters behandeln, vorzugsweise unter Erwärmen.

Bei der basischen Hydrolyse liegt das Molverhältnis von Verbindung der Formel I zu Base typischerweise im Bereich von 0,8 : 1 bis 1 : 10 und ist insbesondere etwa equimolar (d. h. es liegt im Bereich von 0,9:1 bis 1,2:1), jedoch kann auch ein größerer Basenüberschuss, z. B. bis zu 5 Mol je Mol Verbindung I, von Vorteil sein.

Üblicherweise erfolgt die basische Hydrolyse in einem Verdünnungs- bzw. Lösungsmittel. Geeignete Verdünnungs- bzw. Lösungsmittel sind neben Wasser auch organische Lösungsmittel, die gegenüber Alkali stabil sind, sowie deren Mischungen mit Wasser. Beispiele für Alkali-stabile organische Lösungsmittel sind insbesondere die vorgenannten C₁-C₄-Alkanole sowie die vorgenannten acyclischen und die cyclischen Ether. Vorzugsweise führt man die Hydrolyse in wässriger Phase, d. h. in Wasser oder einer Mischung aus Wasser mit einem der vorgenannten organischen Lösungsmittel durch, wobei der Gehalt an organischem Lösungsmittel in der wässrigen Phase in der Regel 30 Vol.-%, bezogen auf die Gesamtmenge an Wasser und organischem Lösungsmittel typischerweise nicht überschreitet.

Vorzugsweise führt man die basische Hydrolyse bei Temperaturen von 20 bis 100 °C durch. In der Regel ist die obere Temperaturgrenze der Siedepunkt des verwendeten Lösungsmittels bei druckloser Reaktionsführung. Vorzugsweise wird man eine Reaktionstemperatur von 100 °C und insbesondere 90 °C nicht überschreiten. Als besonders vorteilhaft hat es sich erwiesen, bei einer Temperatur oberhalb des Siedepunkts der Alkoholkomponente des Esters zu arbeiten. Beispielsweise wird man ausgehend von einer Verbindung der allgemeinen Formel I, worin R¹ für Ethyl steht, die Hydrolyse bevorzugt bei einer Temperatur von wenigstens 80 °C, z. B. im Bereich von 80 bis 100 °C durchführen. Die Reaktionsdauer ist hierbei von der Reaktionstemperatur, der Konzentration und der Stabilität der jeweiligen Esterbindung abhängig. Im Allgemeinen werden die Reaktionsbedingungen so gewählt, dass die Reaktionszeit im Bereich von 1 bis 12 h, insbesondere im Bereich von 2 bis 8 h, liegt.

Die saure Hydrolyse der Verbindung I kann in Analogie zu bekannten sauren Esterhydrolysen durchgeführt werden, d. h. in Gegenwart katalytischer oder stöchiometrischer Mengen einer Säure und Wasser (siehe z. B. J. March, Advanced Organic Chemistry, 2nd Ed., 334-338, McGraw-Hill, 1977 und dort zitierte Literatur). Häufig wird man die Umsetzung in einer Mischung aus Wasser und einem aprotischen organischen Lösungsmittel, beispielsweise einem Ether wie zuvor genannt, durchführen. Beispiele für Säuren sind Halogenwasserstoffsäuren, Schwefelsäure, organische Sulfonsäuren wie p-Toluolsulfonsäure, Methansulfonsäure, Phosphorsäure sowie saure lonentauscherharze und dergleichen.

Geeignete Hydrolysekatalysatoren sind weiterhin Alkalimetalliodide, wie Lithiumiodid, Trimethyliodsilan oder Mischungen von Trimethylchlorsilan mit Alkalimetalliodiden wie Lithium-, Natrium- oder Kaliumiodid.

Die Isolierung der Säure IV erfolgt dann durch übliche Trennverfahren, wie beispielsweise Fällung durch Einstellen des pH-Werts oder Extraktion.

Die erfindungsgemäßen Verbindungen der allgemeinen Formel I eignen sich vorteilhaft zur Synthese einer Vielzahl als Wirkstoff interessanter Verbindungen, wie beispielsweise zur Herstellung von 3-Difluormethylpyrazol-4-carboxamiden der im Folgenden definierten Formel V: 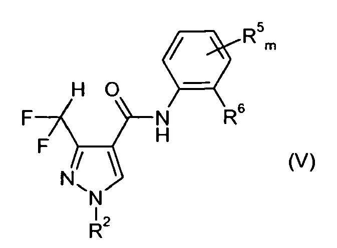 worin
- R²: die zuvor gegebene Bedeutung besitzt;
- R⁵: ausgewählt ist unter Halogen, C₁-C₆-Alkyl, C₁-C₆-Alkoxy, C₁-C₆-Alkylthio, C₁-C₆-Haloalkyl, C₁-C₆-Haloalkoxy oder C₁-C₆-Haloalkylthio;
- m: für 0, 1, 2, 3 oder 4 und insbesondere für 0 oder 1 steht;
- R⁶: ausgewählt ist unter C₁-C₈-Alkyl, C₂-C₆-Alkenyl, C₂-C₆-Alkinyl, C₁-C₆-Alkoxy, C₂-C₆-Alkenyloxy, C₂-C₆-Alkinyloxy, wobei die 6 vorgenannten Reste unsubstituiert sind oder teilweise oder vollständig halogeniert sein können und/oder 1, 2, 3, 4 oder 5 Substituenten R^{ay} tragen können, wobei R^{ay} unabhängig voneinander ausgewählt sind unter Cyano, Nitro, Hydroxy, Mercapto, Amino, Carboxyl, C₁-C₆-Alkoxy, C₂-C₆-Alkenyloxy, C₂-C₆-Alkinyl-oxy, C₁-C₆-Haloalkoxy, C₁-C₆-Alkylthio, C₁-C₆-Alkylamino, Di(C₁-C₆-alkyl)amino, C₁-C₆-Alkylsulfonyl, C₁-C₆-Alkylsulfoxyl, Formyl, C₁-C₆-Alkylcarbonyl, C₁-C₆-Alkoxycarbonyl, Formyloxy und C₁-C₆-Alkylcarbonyloxy;
C₃-C₁₄-Cycloalkyl oder Phenyl, die unsubstituiert sind oder 1, 2, 3, 4 oder 5 Reste R^{ax} substituiert sein können, wobei R^{ax} unabhängig voneinander ausgewählt sind unter Halogen, Cyano, Nitro, Hydroxy, Mercapto, Amino, Carboxyl, C₁-C₆-Alkyl, C₁-C₆-Haloalkyl, C₃-C₆-Cycloalkyl, C₁-C₆-Alkoxy, C₂-C₆-Alkenyloxy, C₂-C₆-Alkinyloxy, C₁-C₆-Haloalkoxy, C₁-C₆-Alkylthio, C₁-C₆-Alkylamino, Di(C₁-C₆-alkyl)amino, C₁-C₆-Alkylsulfonyl, C₁-C₆-Alkylsulfoxyl, Formyl, C₁-C₆-Alkylcarbonyl, C₁-C₆-Alkoxycarbonyl, Formyloxy und C₁-C₆-Alkylcarbonyloxy.

In Formel V ist R⁶ vorzugsweise ausgewählt unter C₁-C₈-Alkyl, C₁-C₈-Haloalkyl, C₁-C₆-Alkoxy, C₁-C₆-Haloalkoxy, Phenyl und Cyclopropyl, wobei Phenyl und Cyclopropyl in der zuvor genannten Weise substituiert sein können. Gemäß einer besonders bevorzugten Ausführungsform steht R⁶ für Phenyl, das gegebenenfalls durch 1, 2, 3, 4 oder 5 Reste R^{ax} substituiert sein kann. R^{ax} ist insbesondere ausgewählt unter Halogen, C₁-C₆-Alkoxy, C₁-C₆-Alkylthio, C₁-C₆-Alkylsulfonyl, C₁-C₆-Alkylsulfoxyl, C₁-C₆-Haloalkylthio, C₁-C₆-Haloalkylsulfonyl und C₁-C₆-Haloalkylsulfoxyl. R⁵ steht insbesondere für Halogen.

Das Verfahren umfasst die Bereitstellung der Pyrazolcarboxylats der Formel I nach dem hier beschriebenen Verfahren und dessen Umsetzung mit einer Aminoverbindung der Formel VI 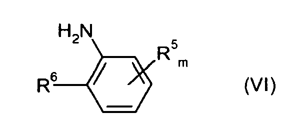 worin m, R⁵ und R⁶ die zuvor genannten Bedeutungen haben; oder
die Bereitstellung der Pyrazol-4-carbonsäure der Formel IV nach den hier beschriebenen Verfahren, gegebenenfalls die Überführung der Pyrazol-4-carbonsäure IV in ihr Carbonsäurehalogenid IVa 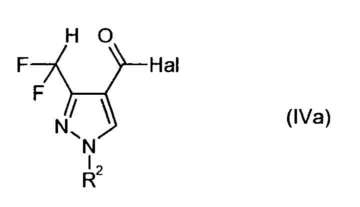 worin R² die zuvor genannte Bedeutung hat und Hal für Halogen, insbesondere für Chlor steht, und die anschließende Umsetzung der Pyrazol-4-carbonsäure der Formel IV oder ihres Carbonsäurehalogenids IVa mit einer Aminoverbindung der Formel VI.

Geeignete Methoden zur Herstellung von Aniliden durch Umsetzung von Carbonsäuren oder Carbonsäureestern mit aromatischen Aminen sind dem Fachmann bekannt, z. B. aus dem eingangs zitierten Stand der Technik sowie aus J. March, Advanced Organic Chemistry, 2nd Ed., 382 f, McGraw-Hill, 1977 und Organikum, 21. Auflage Wiley-VCH, Weinheim 2001, S. 481-484 und dort zitierte Literatur, und können auf die erfindungsgemäße Herstellung der Verbindungen V in analoger Weise übertragen werden.

Beispielsweise kann man Pyrazolcarboxylate der Formel I, insbesondere solche mit R¹ = Methyl oder Ethyl, direkt mit der Anilinverbindung VI im Sinne einer Aminolyse eines Carbonsäureesters umsetzen. Alternativ kann man die Pyrazolcarbonsäure der Formel IV direkt mit der Anilinverbindung VI im Sinne einer Aminolyse einer Carbonsäure umsetzen.

Häufig wird man jedoch so vorgehen, dass man die Pyrazolcarbonsäure der Formel IV zunächst in ihr Säurehalogenid, z. B. ihr Säurechlorid überführt und anschließend das Säurehalogenid IVa mit der Anilinverbindung VI umsetzt.

In Abhängigkeit des ausgewählten Synthesewegs kann die Kupplungsreaktion von Carbonsäure IV bzw. Carbonsäurederivat I oder IVa und Anilinderivat VI gegebenenfalls in Gegenwart von Katalysatoren, Kondensationsmitteln, Säurebindungsmitteln und/oder unter Wasserabscheidung, beispielsweise durch azeotrope Destillation, durchgeführt werden.

Im Allgemeinen erfolgt die Umsetzung des Carbonsäurehalogenids IVa mit der Anilinverbindung VI in einem inerten Lösungsmittel. Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Methylenchlorid, Dimethylsulfoxid und Dimethylformamid, besonders bevorzugt Toluol, Methylenchlorid und Tetrahydrofuran. Es können auch Gemische der genannten Lösungsmittel verwendet werden.

Die Umsetzung von IVa mit VI erfolgt üblicherweise in Gegenwart einer Base. Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, außerdem organische Basen, z. B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin verwendet.

Die Basen werden im Allgemeinen in äquimolaren Mengen, bezogen auf die Verbindung IVa, eingesetzt. Sie können aber auch in einem Überschuss von 5 Mol-% bis 30 Mol-%, vorzugsweise 5 Mol-% bis 10 Mol-%, oder - im Falle der Verwendung von tertiären Aminen - gegebenenfalls als Lösungsmittel verwendet werden.

Die Edukte IVa und VI werden im Allgemeinen in äquimolaren Mengen miteinander umgesetzt. Es kann für die Ausbeute vorteilhaft sein, IVa in einem Überschuss von 1 Mol-% bis 20 Mol-%, vorzugsweise 1 Mol-% bis 10 Mol-%, bezogen auf VI einzusetzen.

Diese Umsetzung der Pyrazolcarbonsäurehalogenide IVa mit den Anilinverbindungen VI erfolgt üblicherweise bei Temperaturen von -20 °C bis 100 °C, vorzugsweise 0 °C bis 50 °C.

Die Umsetzung der Pyrazolcarbonsäuren IV mit den Anilinverbindungen VI erfolgt üblicherweise in Gegenwart eines Dehydratisierungsmittels. Als Dehydratisierungsmittel kommen beispielsweise 1,1'-Carbonyldiimidazol, Bis(2-oxo-3-oxazolidinyl)phosphorylchlorid, Carbodiimide wie N,N'-Dicyclohexylcarbodiimid, N-(3-Dimethylaminopropyl)-N'-ethylcarbodiimid, Phosphoniumsalze wie (Benzotriazol-1-yloxy)tris(dimethylamino)-phosphoniumhexafluorphosphat, Bromtripyrrolidino-phosphoniumhexafluorphosphat, Bromtris(dimethylamino)phosphoniumhexafluor-phosphat, Chlortripyrrolidinophosphoniumhexafluorphosphat, Uronium- und Thiuroniumsalze wie O-(Benzotriazol-1-yl)-N,N,N',N'-tetramethyluroniumhexafluorphosphat, O-(7-Azabenzotriazol-1-yl)-N,N,N',N'-tetramethyluroniumhexafluorphosphat, S-(1-Oxido-2-pyridyl)-N,N,N',N'-tetramethylthiuroniumtetrafluorborat, O-(2-Oxo-1(2H)pyridyl)-N,N,N',N'-tetramethyluroniumtetrafluorborat, O-[(Ethoxycarbonyl)cyanomethylenamino]-N,N,N',N'-tetramethyluroniumtetrafluorborat, Carbeniumsalze wie (Benzotriazol-1-yloxy)-dipyrrolidinocarbeniumhexafluorphosphat, (Benzotriazol-1-yloxy)dipiperidinocarbeniumhexafluorphosphat, O-(3,4-Dihydro-4-oxo-1,2,3-benzotriazin-3-yl)-N,N,N',N'-tetramethyluroniumtetrafluorborat, Chlor-N',N'-bis(tetramethylen)formamidiniumtetrafluorborat, Chlordipyrrolidinocarbeniumhexafluorphosphat, Chlor-N,N,N',N'-bis(pentamethylen)formamidiniumtetrafluorborat, Imidazoliumsalze wie 2-Chlor-1,3-dimethylimidazolidiniumtetrafluorborat, vorzugsweise 1,1'-Carbonyldiimidazol, Bis(2-oxo-3-oxazolidinyl)phosphorylchlorid, N,N'-Dicyclohexyl-carbodiimid und N-(3-Dimethylaminopropyl)-N'-ethylcarbodiimid, in Betracht.

Sofern die Umsetzung der Pyrazolcarbonsäuren IV mit den Anilinverbindungen VI in Gegenwart eines Dehydratisierungsmittels durchgeführt wird, verwendet man vorzugsweise eine organische Base. Als organische Basen kommen beispielsweise tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin verwendet. Die Basen werden im Allgemeinen im Überschuss von 10 Mol-% bis 200 Mol-%, vorzugsweise von 50 Mol-% bis 150 Mol-%, bezogen auf die Verbindung IV, eingesetzt.

Die Edukte IV und VI werden im Allgemeinen in etwa äquimolaren Mengen miteinander umgesetzt. Es kann für die Ausbeute vorteilhaft sein, eine der Verbindungen in einem Überschuss von 1 Mol-% bis 20 Mol-%, vorzugsweise 1 Mol-% bis 10 Mol-%, einzusetzen. Die Dehydratisierungsmittel werden im Allgemeinen im Überschuss von 5 Mol-% bis 100 Mol-%, vorzugsweise 5 Mol-% bis 60 Mol-%, bezogen auf die Verbindung IV eingesetzt.

Die Umsetzung von IV mit VI erfolgt üblicherweise in einem Lösungsmittel. Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Methylenchlorid, Toluol und Tetrahydrofuran. Es können auch Gemische der genannten Lösungsmittel verwendet werden.

Im Folgenden wird die Herstellung Difluormethyl-substituierter Pyrazol-4-ylcarbonsäureestern und deren Weiterverarbeitung anhand von Beispielen erläutert..

Herstellungsbeispiel 1: Herstellung von 2-Ethoxymethylen-4,4,4-trifluor-3-oxobuttersäureethylester

In einem 500 mL Vierhalskolben mit Rührer vermischte man 78,3 g (0,425 Mol) Trifluoracetoacetat (4,4,4-Trifluor-3-oxobuttersäureethylester), 103,3 g (0,638 Mol) Triethylorthoformiat und 130,0 g (1,275 Mol) Essigsäureanhydrid und erwärmte 6 Stunden auf 120 °C. Anschließend wurden bei Normaldruck zuerst die niedrig siedenden Bestandteile abgetrennt und im Anschluss das Produkt im Vakuum über eine Kolonne destilliert. Man erhielt so 91,8 g (Ausbeute 90 %) der Titelverbindung als farblose Flüssigkeit mit einer Reinheit > 98 %.

### Herstellungsbeispiel 2: Herstellung von 2-lsopropoxymethylen-4,4,4-trifluor-3-oxobuttersäureisopropylester

Die Herstellung erfolgte analog Herstellungsbeispiel 1 unter Verwendung von 4,4,4-Trifluor-3-oxobuttersäureisopropylester und Triisopropylorthoformiat.

### Beispiel 1: Herstellung von 3-Difluormethyl-1-methylpyrazol-4-carbonsäureethylester

In einem mit Magnetrührer und Thermometer ausgestatteten 500 ml Dreihalskolben wurden Magnesiumspäne (4,9 g, 0,20 Mol), Trimethylsilylchlorid (TMS-Cl: 21,8 g, 0,20 mmol) und wasserfreies Dimethylformamid (DMF, 240 ml) vorgelegt. Nach Aktivierung des Magnesiums durch Ultraschall wurde im Eisbad 2-Ethoxymethylen-4,4,4-trifluor-3-oxobuttersäureethylester (25,3 g, 0,10 Mol) über einen Zeitraum von 30 min zugegeben, wobei die Reaktionstemperatur in einem Bereich von 0 bis 10 °C gehalten wurde. Nach weiteren 60 min wurde das überschüssige Trimethylsilylchlorid unter vermindertem Druck entfernt. In einem zweiten 500 ml Dreihalskolben wurde bei -50 °C eine wässrige Methylhydrazin-Lösung (37 %, 20,8 g, 0,12 Mol) und Ethanol (320 ml) vorgelegt. Hierzu gab man über einen Zeitraum von 60 min die gekühlte Reaktionslösung der ersten Umsetzung, wobei man die Kühlung beibehielt. Nach weiteren 2 Stunden bei -50 °C ließ man die Reaktionsmischung auf Raumtemperatur erwärmen und rührte weitere 10 Stunden. Die Reaktionsmischung enthielt laut GC-Analyse 3-Difluormethyl-1-methylpyrazol-4-carbonsäureethylester (Isomer a) im Gemisch mit 2-Difluormethyl-1-methylpyrazol-3-carbonsäureethylester (Isomer b) mit einem Isomerenverhältnis a : b von 82 : 18.

Anschließend wurde die Reaktionsmischung unter vermindertem Druck eingeengt. Der Rückstand wurde in 100 ml Ethylacetat aufgenommen und dreimal mit gesättigter, wässriger Kochsalzlösung, deren pH-Wert durch Zugabe konz. Salzsäure auf pH 2 eingestellt worden war, gewaschen. Die organische Phase wurde unter vermindertem Druck vom Lösungsmittel befreit. Der feste Rückstand wurde aus Hexan umkristallisiert. 3-Difluormethyl-1-methylpyrazol-4-carbonsäureethylester wurde als farbloses kristallines Pulver (15,3 g, 70 % Ausbeute, 95 % Reinheit, Isomerenreinheit a : b = 94 : 6) erhalten.

¹H-NMR (d⁶-DMSO, 400 MHz): δ = 1,27 (t, 3 H, J = 7,1 Hz), 3,92 (s, 3 H), 4,23 (q, 2 H, J = 7,1 Hz), 7,21 (t, 1 H, J=53 Hz), 8,41 ppm (s, 1 H).

### Beispiel 1 a-1 e: Versuche zur Herstellung von 3-Difluormethyl-1-methylpyrazol-4-carbonsäureethylester in verschiedenen Lösungsmitteln bei der Umsetzung der Zwischenverbindung A mit Methylhydrazin

Analog Beispiel 1 wurden 2-Ethoxymethylen-4,4,4-trifluor-3-oxobuttersäureethylester, Mg und TMS-Cl in DMF umgesetzt und der Überschuss an TMS-Cl wurde unter vermindertem Druck entfernt. Nachfolgend wurde der Ansatz mit wässriger Methylhydrazin-Lösung (37 %) in unterschiedlichen Lösungsmitteln zum 3-Difluormethyl-1-methylpyrazol-4-carbonsäureethylester umgesetzt. Die folgende Tabelle 1 zeigt die erzielten Isomerengemische je nach Lösungsmittel und Mengenverhältnis der Reagenzien.

**Tabelle 1:**

| Beispiel | Lösungsmittel Typ | Lösungsmittel Menge | Isomer a [%] | Isomer b [%] |
|---|---|---|---|---|
| 1a | Methanol | 480 ml | 86 | 14 |
| 1b | Ethanol | 320 ml | 84 | 16 |
| 1c | Acetonitril | 320 ml | 84 | 16 |
| 1d | Toluol | 480 ml | 80 | 20 |
| 1e | Tetrahydrofuran | 480 ml | 80 | 20 |

### Beispiel 2: Herstellung von 3-Difluormethyl-1-methylpyrazol-4-carbonsäure

Die Herstellung erfolgte analog Beispiel 1, wobei man im Unterschied zu Beispiel 1 anstelle einer Umkristallisation aus Hexan den nach wässrig extraktiver Aufarbeitung und Entfernen des Ethylacetats erhaltenen festen Rückstand wie folgt weiterverarbeitete:
Man versetzte den festen Rückstand mit 16 g einer 50 gew.-%igen wässrigen Natronlauge und 100 ml Ethanol und erhitzte das Gemisch unter Rühren 4 h zum Rückfluss. Anschließend wurde das Lösungsmittel unter vermindertem Druck entfernt und der erhaltene wässrige Rückstand wurde mit 10 %iger Salzsäure auf pH 1 gebracht. Hierbei fiel die Dicarbonsäure als Feststoff an, der mittels Filtration isoliert wurde. Auf diese Weise erhielt man 3-Difluormethyl-1-methylpyrazol-4-carbonsäure als hellbraunes Pulver.

¹H-NMR (d⁶-DMSO, 400 MHz): δ = 3,92 (s, 3 H), 7,21 (t, 1 H, J=53 Hz), 8,34 ppm (s, 1 H).

### Beispiel 3: Herstellung von 3-Difluormethyl-1-methylpyrazol-4-carbonsäureethylester

In einem mit Magnetrührer und Thermometer ausgestatteten 500 ml Dreihalskolben wurden Magnesiumspäne (4,9 g, 0,20 Mol) vorgelegt. Nach Aktivierung des Magnesiums durch Anätzen mit Jod und anschließender Zugabe von wasserfreiem N-Methylpyrrolidon (NMP, 240 ml) und Trimethylsilylchlorid (21,8 g, 0,20 mmol) wurde 2-Ethoxymethylen-4,4,4-trifluor-3-oxobuttersäureethylester (25,3 g, 0,10 Mol) über einen Zeitraum von 30 min bei einer Temperatur im Bereich von 30 bis 40 °C zugegeben. Nach weiteren 120 min wurde das überschüssige Trimethylsilylchlorid unter vermindertem Druck entfernt. In einem zweiten 500 ml Dreihalskolben wurde bei -50 °C eine wässrige Methylhydrazin-Lösung (37 %, 20,8 g, 0,12 Mol) und Ethanol (320 ml) vorgelegt. Hierzu gab man über einen Zeitraum von 60 min die gekühlte Reaktionslösung der ersten Umsetzung, wobei man die Kühlung beibehielt. Nach weiteren 2 Stunden bei -50 °C ließ man die Reaktionsmischung auf Raumtemperatur erwärmen und rührte weitere 10 Stunden. Die Reaktionsmischung enthielt laut GC-Analyse 3-Difluormethyl-1-methylpyrazol-4-carbonsäureethylester (Isomer a) im Gemisch mit 2-Difluormethyl-1-methylpyrazol-3-carbonsäureethylester (Isomer b) mit einem Isomerenverhältnis a : b von 84 : 16. Anschließend wurde die Reaktionsmischung unter vermindertem Druck eingeengt.

Als Produkt erhielt man eine Lösung von 3-Difluormethyl-1-methylpyrazol-4-carbonsäureethylester in NMP, das durch Extraktion oder Kristallisation analog Beispiel 1 gereinigt oder analog Beispiel 2 durch Kochen mit Natronlauge zur Säure verseift werden kann.

### Beispiel 4: Herstellung von 3-Difluormethyl-1-methylpyrazol-4-carbonsäure

In einem mit Magnetrührer und Thermometer ausgestatteten 500 ml Dreihalskolben wurden Magnesiumspäne (4,9 g, 0,20 Mol), Trimethylsilylchlorid (32,6 g, 0,30 mmol) und wasserfreies 1,3-Dimethyl-2-imidazolidinon (DMI, 160 ml) vorgelegt. Nach Aktivierung des Magnesiums durch Jod wurde im Eisbad 2-Methoxymethylen-4,4,4-trifluor-3-oxobuttersäuremethylester (21,3 g, 0,10 Mol) über einen Zeitraum von 30 min zugegeben, wobei die Reaktionstemperatur in einem Bereich von 20 bis 30 °C gehalten wurde. Nach weiteren 60 min bei Raumtemperatur wurde das überschüssige Trimethylsilylchlorid unter vermindertem Druck entfernt. In einem zweiten 500 ml Dreihalskolben wurde bei -50 °C eine wässrige Methylhydrazin-Lösung (37 %, 14,8 g, 0,12 Mol) und Methanol (320 ml) vorgelegt. Hierzu gab man über einen Zeitraum von 60 min die gekühlte Reaktionslösung der ersten Umsetzung, wobei man die Kühlung beibehielt. Nach weiteren 2 Stunden bei -50 °C ließ man die Reaktionsmischung auf Raumtemperatur erwärmen und rührte weitere 10 Stunden. Die Reaktionsmischung enthielt laut GC-Analyse 3-Difluormethyl-1-methylpyrazol-4-carbonsäuremethylester (Isomer a) im Gemisch mit 2-Difluormethyl-1-methylpyrazol-3-carbonsäuremethylester (Isomer b) mit einem Isomerenverhältnis a : b von 90 : 10. Anschließend wurde die Reaktionsmischung unter vermindertem Druck eingeengt. Der Rückstand wurde mit 120 g 10%iger Natronlauge versetzt und 4 h bei 100 °C gerührt. Nach Ansäuern der wässrigen Lösung mit Salzsäure auf pH 1 und mehrfache Extraktion mit MTBE erhielt man eine organische Lösung, die 13,4 g der Titelverbindung enthielt (Ausbeute Isomer a: 71 %). Die Titelverbindung wurde durch Kristallisation als hellbrauner Feststoff isoliert.

### Beispiel 5: Herstellung von 3-Difluormethyl-1-methylpyrazol-4-carbonsäure-isopropylester

In einem mit Magnetrührer und Thermometer ausgestatteten 500 ml Dreihalskolben wurden Magnesiumspäne (4,9 g, 0,20 Mol), Trimethylsilylchlorid (21,8 g, 0,20 mmol) und wasserfreies Dimethylformamid (DMF, 250 ml) vorgelegt. Nach Aktivierung des Magnesiums durch Ultraschall wurde im Eisbad 2-lsopropoxymethylen-4,4,4-trifluor-3-oxobuttersäureisopropylester (26,8 g, 0,10 Mol) über einen Zeitraum von 30 min zugegeben; wobei die Reaktionstemperatur in einem Bereich von 0 bis 10 °C gehalten wurde. Nach weiteren 60 min wurde das überschüssige Trimethylsilylchlorid unter vermindertem Druck entfernt. In einem zweiten 500 ml Dreihalskolben wurde bei -50 °C eine wässrige Methylhydrazin-Lösung (37 %, 20,8 g, 0,12 Mol) und Ethanol (320 ml) vorgelegt. Hierzu gab man über einen Zeitraum von 60 min die gekühlte Reaktionslösung der ersten Umsetzung, wobei man die Kühlung beibehielt. Nach weiteren 2 Stunden bei -50 °C ließ man die Reaktionsmischung auf Raumtemperatur erwärmen und rührte weitere 10 Stunden. Die Reaktionsmischung enthielt laut GC-Analyse 3-Difluormethyl-1-methylpyrazol-4-carbonsäureisopropylester (Isomer a) im Gemisch mit 2-Difluormethyl-1-methylpyrazol-3-carbonsäureisopropylester (Isomer b) mit einem Isomerenverhältnis a : b von 83 : 17.

### Beispiel 6: 3-Difluormethyl-1-methylpyrazol-4-carbonsäurechlorid

Man erwärmte eine Lösung von 293 g 3-Difluormethyl-1-methylpyrazol-4-carbonsäure, hergestellt analog Beispiel 2, in 700 g Toluol auf 90 °C und gab hierzu innerhalb 3,5 h 260 g Thionylchlorid. Man ließ abkühlen und engte die Mischung im Vakuum ein, versetzte den Rückstand mit 100 ml Toluol und engte erneut im Vakuum ein. Der Rückstand wurde über eine Füllkörperkolonne bei einem Druck von 0,8 mbar und einer Kopftemperatur von 109 °C destilliert, wobei man 298,4 g des Säurechlorids mit einer Reinheit von 99 % erhielt (Ausbeute 92,1 %).

### Beispiel 7: 3-Difluormethyl-1-methylpyrazol-4-carbonsäure-N-(3,4'-dichlor-5-fluorbiphenyl-2-yl)amid

In einem 2-L Vierhalskolben mit Rührer und Tropftrichter löste man 208 g (0,788 Mol) 2-Amino-3',4'-dichlor-5-fluorbiphenyl (Reinheit 97 %) und 82,1 g (1,04 Mol) Pyridin in 1100 ml trockenem Toluol, erwärmte auf 45 °C und gab hierzu innerhalb von 30 min 155 g (0,788 Mol) des nach Beispiel 6 hergestellten 3-Difluormethyl-1-methylpyrazol-4-carbonsäurechlorids über den Tropftrichter zu. Man spülte den Tropftrichter mit einer geringen Menge Toluol nach und rührte 1 h bei 75 °C. Anschließend extrahierte man in der Wärme nacheinander mit 270 ml einer 5 gew.-%igen wässrigen Salzsäure, 270 ml einer 10 gew.-%igen wässrigen Natriumhydrogencarbonat-Lösung und 270 ml entionisiertem Wasser. Die organische Phase wurde unter Rühren auf Raumtemperatur gekühlt. Hierbei fiel das Produkt als Feststoff aus, der über einen Glasfilter abgesaugt und mit etwas kaltem Toluol (0 °C) nachgewaschen wurde. Der Feststoff wurde anschließend im Vakuum getrocknet. Man erhielt so 264 g der Titelverbindung als weißen Feststoff mit einer Reinheit > 99 %.

In zu Beispiel 7 analoger Weise wurden die in Tabelle 2 angegebenen Pyrazol-4-carbonsäureanilide der allgemeinen Formel V (R² = CH₃) hergestellt:

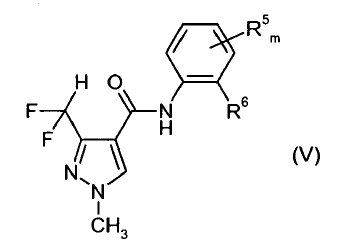

**Tabelle 2**

| Beispiel | R⁵ₘ | R⁶ | Ausbeute [%] |
|---|---|---|---|
| 8 | 5-F | 3,4-Dichlorphenyl | 88 |
| 9 | -- | 1,1,2,3,3,3-Hexafluorpropoxy | 89 |
| 10 | -- | 1,1,2,2-Tetrafluorethoxy | 92 |
| 11 | -- | 4-(Methoxyiminomethyl)-3-fluorphenyl | 82 |
| 12 | -- | 1,3-Dimethylbutyl | 86 |
| 13 | 5-F | 4-Chlor-3-fluorphenyl | 45 |
| 14 | 5-F | 3-Fluor-4-methylphenyl | 90 |
| 15 | -- | 2-(Cyclopropyl)-cyclopropyl | 81 |
| 16 | -- | 2,4-Difluorphenyl | 96 |
| 17 | -- | 2,5-Difluorphenyl | 80 |
| 18 | -- | 2,4-Dichlorphenyl | 88 |
| 19 | -- | 2,5-Dichlorphenyl | 82 |
| 20 | -- | 3,5-Difluorphenyl | 79 |
| 21 | -- | 3,5-Dichlorphenyl | 95 |
| 22 | -- | 3-Fluorphenyl | 72 |
| 23 | -- | 3-Chlorphenyl | 79 |
| 24 | -- | 2-Fluorphenyl | 63 |
| 25 | -- | 2-Chlorphenyl | 69 |
| 26 | -- | 3,4,5-Trifluorphenyl | 93 |
| 27 | -- | 2,4,5-Trifluorphenyl | 89 |

## Patentansprüche

1. Verfahren zur Herstellung von Difluormethyl-substituierten Pyrazol-4-ylcarboxylaten der allgemeinen Formel (I), 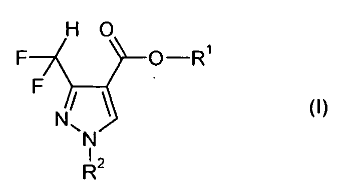 worin
R¹ für C₁-C₈-Alkyl, C₃-C₈-Cycloalkyl, C₁-C₄-Alkoxy-C₁-C₄-alkyl, C₃-C₈-Cycloalkoxy-C₁-C₄-alkyl, C₂-C₈-Alkenyl oder für gegebenenfalls durch 1, 2 oder 3 unabhängig voneinander unter C₁-C₄-Alkyl, C₁-C₄-Alkoxy oder Nitro ausgewählten Substituenten R^{y1} substituiertes Benzyl steht; und
R² für Wasserstoff, C₁-C₄-Alkyl, Benzyl oder Phenyl steht, wobei die beiden letztgenannten Substituenten unsubstituiert oder gegebenenfalls durch 1, 2 oder 3 unabhängig voneinander unter Halogen, CN, Nitro, C₁-C₄-Alkyl, C₁-C₄-Haloalkyl, C₁-C₄-Alkoxy oder C₁-C₄-Haloalkoxy ausgewählte Substituenten R^{y2} substituiert sein können;
wobei man
a) eine Verbindung der allgemeinen Formel (II) 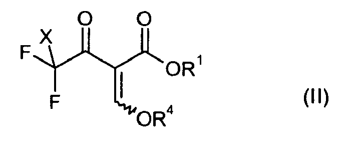 worin X für Fluor, Chlor oder Brom steht, R¹ eine der zuvor gegebenen Bedeutungen besitzt und R⁴ für C₁-C₈-Alkyl, C₃-C₈-Cycloalkyl, C₂-C₈-Alkenyl, Benzyl oder Phenyl steht,
mit einer Silanverbindung der allgemeinen Formel R³ₙSiCl₍₄₋ₙ₎,
worin n für 1, 2 oder 3 steht und die Substituenten R³ unabhängig voneinander ausgewählt sind unter C₁-C₈-Alkyl oder Phenyl, und
mit einem Metall umsetzt, das unter den Metallen der Gruppen 1, 2, 3, 4 und 12 des Periodensystems ausgewählt ist und ein Redoxpotential von weniger als -0,7 V, bezogen auf eine Normalwasserstoffelektrode (bei 25 °C und 101,325 kPa), aufweist; und
b) das Reaktionsgemisch aus Schritt a) mit einer Verbindung der allgemeinen Formel (III)
R²HN-NH₂ (III)
umsetzt, worin R² eine der zuvor gegebenen Bedeutungen besitzt.

2. Verfahren nach Anspruch 1, wobei das Metall Magnesium ist.

3. Verfahren nach einem der vorhergehenden Ansprüche, wobei man eine Silanverbindung der allgemeinen Formel R³ₙSiCl₍₄₋ₙ₎ einsetzt, worin n für 2 oder 3 steht.

4. Verfahren nach einem der vorhergehenden Ansprüche, wobei in der Silanverbindung die Substituenten R³ unabhängig voneinander ausgewählt sind unter C₁-C₄-Alkyl.

5. Verfahren nach Anspruch 3 oder 4, wobei die Silanverbindung ausgewählt ist unter Trimethylsilylchlorid, Ethyldimethylsilylchlorid, Dimethylpropylsilylchlorid, Dimethylisopropylsilylchlorid, n-Butyldimethylsilylchlorid, 2-Butyldimethylsilylchlorid, (2-Methylpropyl)dimethylsilylchlorid, Dimethyldichlorsilan, Diethyldichlorsilan und tert.-Butyldimethylsilylchlorid.

6. Verfahren nach einem der vorhergehenden Ansprüche, worin R⁴ in Formel II ausgewählt ist unter C₁-C₄-Alkyl oder Benzyl.

7. Verfahren nach einem der vorhergehenden Ansprüche, wobei die Umsetzung in Schritt a) im Wesentlichen wasserfrei erfolgt.

8. Verfahren nach Anspruch 7, wobei man die Umsetzung in Schritt a) in einem unter N-C₁-C₄-Alkyllactamen, N-Di(C₁-C₄-alkyl)amiden aliphatischen C₁-C₄-Carbonsäuren, N,N,N',N'-Tetra(C₁-C₄-alkyl)alkylharnstoffen, 1,3-Di(C₁-C₄-alkyl)hexahydropyrimidin-2-on und 1,3-Di(C₁-C₄-alkyl)imidazolin-2-on ausgewählten Lösungsmittel durchführt.

9. Verfahren nach einem der vorhergehenden Ansprüche, wobei man die Umsetzung in Schritt b) in Gegenwart von Wasser durchführt.

10. Verfahren nach Anspruch 9, wobei man die Umsetzung in Schritt b) in einem Gemisch aus C₁-C₄-Alkanol und Wasser durchführt.

11. Verfahren nach einem der vorhergehenden Ansprüche, wobei in den Formeln I und II R¹ für C₁-C₄-Alkyl oder Benzyl steht.

12. Verfahren nach einem der vorhergehenden Ansprüche, wobei die Verbindung der allgemeinen Formel (III) ausgewählt ist unter C₁-C₄-Alkylhydrazinen und Hydrazinhydrat.

13. Verfahren zur Herstellung einer Pyrazol-4-carbonsäure der allgemeinen Formel (IV) 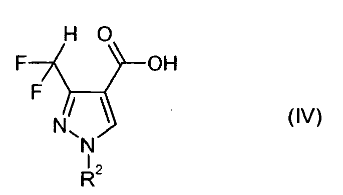 worin R² eine der zuvor gegebenen Bedeutungen besitzt, umfassend
i) die Bereitstellung einer Verbindung der Formel I nach einem Verfahren gemäß einem der Ansprüche 1 bis 10, und
ii) eine Hydrolyse der Verbindung I zur Carbonsäure IV.

14. Verfahren nach Anspruch 13, wobei die basische Hydrolyse in Gegenwart einer wässrigen Alkalimetallhydroxid-Lösung oder Erdalkalimetallhydroxid-Lösung erfolgt.

15. Verfahren zur Herstellung von Pyrazol-4-carbonsäureaniliden der allgemeinen Formel (V), 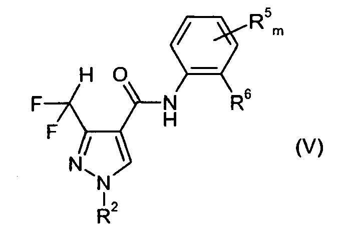 worin
R² die zuvor gegebene Bedeutung besitzt;
R⁵ ausgewählt ist unter Halogen, C₁-C₆-Alkyl, C₁-C₆-Alkoxy, C₁-C₆-Alkylthio, C₁-C₆-Haloalkyl, C₁-C₆-Haloalkoxy oder C₁-C₆-Haloalkylthio;
m für 0, 1, 2, 3 oder 4 steht;
R⁶ ausgewählt ist unter C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, wobei die 6 vorgenannten Reste unsubstituiert sind oder teilweise oder vollständig halogeniert sein können und/oder 1, 2, 3, 4 oder 5 Substituenten R^{ay} tragen können, wobei R^{ay} unabhängig voneinander ausgewählt sind unter Cyano, Nitro, Hydroxy, Mercapto, Amino, Carboxyl, C₁-C₆-Alkoxy, C₂-C₆-Alkenyloxy, C₂-C₆-Alkinyl-oxy, C₁-C₆-Haloalkoxy, C₁-C₆-Alkylthio, C₁-C₆-Alkylamino, Di(C₁-C₆-alkyl)amino, C₁-C₆-Alkylsulfonyl, C₁-C₆-Alkylsulfoxyl, Formyl, C₁-C₆-Alkylcarbonyl, C₁-C₆-Alkoxycarbonyl, Formyloxy und C₁-C₆-Alkylcarbonyloxy;
C₃-C₁₄-Cycloalkyl oder Phenyl, die unsubstituiert sind oder 1, 2, 3, 4 oder 5 Reste R^{ax} substituiert sein können, wobei R^{ax} unabhängig voneinander ausgewählt sind unter Halogen, Cyano, Nitro, Hydroxy, Mercapto, Amino, Carboxyl, C₁-C₆-Alkyl, C₁-C₆-Haloalkyl, C₃-C₆-Cycloalkyl, C₁-C₆-Alkoxy, C₂-C₆-Alkenyloxy, C₂-C₆-Alkinyloxy, C₁-C₆-Haloalkoxy, C₁-C₆-Alkylthio, C₁-C₆-Alkylamino, Di(C₁-C₆-alkyl)amino, C₁-C₆-Alkylsulfonyl, C₁-C₆-Alkylsulfoxyl, Formyl, C₁-C₆-Alkylcarbonyl, C₁-C₆-Alkoxycarbonyl, Formyloxy und C₁-C₆-Alkylcarbonyloxy;
umfassend:
i) die Bereitstellung eines Pyrazolcarboxylats der Formel I durch ein Verfahren gemäß einem der Ansprüche 1 bis 10 und die Umsetzung des Pyrazolcarboxylats der Formel I mit einer Aminoverbindung der Formel VI 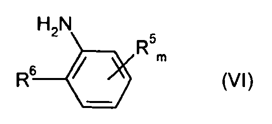 worin m, R⁵ und R⁶ die zuvor genannten Bedeutungen haben;
oder
ii) Bereitstellung einer Pyrazol-4-carbonsäure der Formel IV durch ein Verfahren gemäß Anspruch 11, gegebenenfalls Überführung der Pyrazol-4-carbonsäure IV in ihr Carbonsäurehalogenid und die anschließende Umsetzung der Pyrazol-4-carbonsäure der Formel IV oder ihres Carbonsäurehalogenids mit einer Aminoverbindung der Formel VI.

16. Verbindung der Formel A 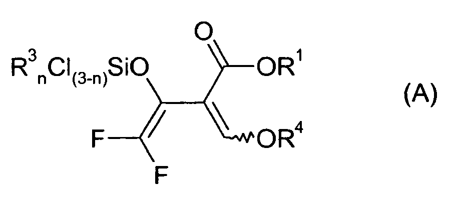 worin R¹ und R⁴ eine der für Formel II angegebenen Bedeutungen aufweisen und R³ unabhängig voneinander ausgewählt sind unter C₁-C₈-Alkyl oder Phenyl.

## Claims

1. A process for preparing difluoromethyl-substituted pyrazol-4-ylcarboxylates of the general formula (I) 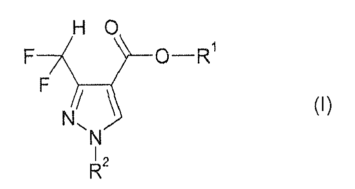 in which
R¹ is C₁-C₈-alkyl, C₃-C₈-cycloalkyl, C₁-C₄-alkoxy-C₁-C₄-alkyl, C₃-C₈-cycloalkoxy-C₁-C₄-alkyl, C₂-C₈-alkenyl or is benzyl which is optionally substituted by 1, 2 or 3 substituents R^{y1} independently of one another selected from the group consisting of C₁-C₄-alkyl, C₁-C₄-alkoxy and nitro; and
R² is hydrogen, C₁-C₄-alkyl, benzyl or phenyl, where the two last-mentioned substituents may be unsubstituted or optionally substituted by 1, 2 or 3 substituents R^{y2} independently of one another selected from the group consisting of halogen, CN, nitro, C₁-C₄-alkyl, C₁-C₄-haloalkyl, C₁-C₄-alkoxy and C₁-C₄-haloalkoxy;
wherein
a) a compound of the general formula (II) 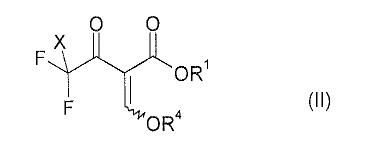 in which X is fluorine, chlorine or bromine, R¹ has one of the meanings given above and R⁴ is C₁-C₈-alkyl, C₃-C₈-cycloalkyl, C₂-C₈-alkenyl, benzyl or phenyl,
is reacted with a silane compound of the general formula R³ₙSiCl₍₄₋ₙ₎
in which n is 1, 2 or 3 and the substituents R³ are independently of one another selected from the group consisting of C₁-C₈-alkyl and phenyl and
with a metal selected from the metals of groups 1, 2, 3, 4 and 12 of the Periodic Table of the Elements having a redox potential of less than -0.7 V, based on a normal hydrogen electrode (at 25°C and 101.325 kPa); and
b) the reaction mixture from step a) is reacted with a compound of the general formula (III) R²HN-NH₂ (III)
in which R² has one of the meanings given above.

2. The process according to claim 1 wherein the metal is magnesium.

3. The process according to either of the preceding claims wherein a silane compound of the general formula R³ₙSiCl₍₄₋ₙ₎ in which n is 2 or 3 is employed.

4. The process according to any of the preceding claims wherein in the silane compound the substituents R³ are independently of one another selected from the group consisting of C₁-C₄-alkyl.

5. The process according to claim 3 or 4 wherein the silane compound is selected from the group consisting of trimethylsilyl chloride, ethyldimethylsilyl chloride, dimethylpropylsilyl chloride, dimethylisopropylsilyl chloride, n-butyldimethylsilyl chloride, 2-butyldimethylsilyl chloride, (2-methylpropyl)dimethylsilyl chloride, dimethyldichlorosilane, diethyldichlorosilane and tert-butyldimethylsilyl chloride.

6. The process according to any of the preceding claims wherein R⁴ in formula II is selected from the group consisting of C₁-C₄-alkyl and benzyl.

7. The process according to any of the preceding claims wherein the reaction in step a) is carried out essentially anhydrously.

8. The process according to claim 7 wherein the reaction in step a) is carried out in a solvent selected from the group consisting of N-C₁-C₄-alkyllactams, N-di(C₁-C₄-alkyl)amides of aliphatic C₁-C₄-carboxylic acids, N,N,N',N'-tetra(C₁-C₄-alkyl)alkylureas, 1,3-di(C₁-C₄-alkyl)hexahydropyrimidin-2-one and 1,3-di(C₁-C₄-alkyl)imidazolin-2-one.

9. The process according to any of the preceding claims wherein the reaction in step b) is carried out in the presence of water.

10. The process according to claim 9 wherein the reaction in step b) is carried out in a mixture of C₁-C₄-alkanol and water.

11. The process according to any of the preceding claims wherein in formulae I and II R¹ is C₁-C₄-alkyl or benzyl.

12. The process according to any of the preceding claims wherein the compound of the general formula (III) is selected from C₁-C₄-alkylhydrazines and hydrazine hydrate.

13. A process for preparing a pyrazole-4-carboxylic acid of the general formula (IV) 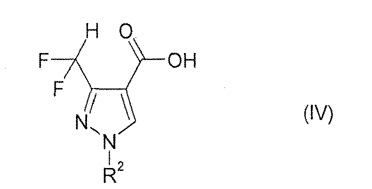 in which R² has one of the meanings given above, comprising
i) the provision of a compound of the formula I according to a process according to any of claims 1 to 10, and
ii) a hydrolysis of the compound I to give the carboxylic acid IV.

14. The process according to claim 13 wherein the basic hydrolysis is carried out in the presence of an aqueous alkali metal hydroxide solution or alkaline earth metal hydroxide solution.

15. A process for preparing pyrazole-4-carboxanilides of the general formula (V) 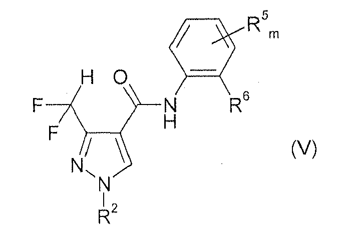 in which
R² has the meaning given above;
R⁵ is selected from the group consisting of halogen, C₁-C₆-alkyl, C₁-C₆-alkoxy, C₁-C₆-alkylthio, C₁-C₆-haloalkyl, C₁-C₆-haloalkoxy and C₁-C₆-haloalkylthio;
m is 0, 1, 2, 3 or 4;
R⁶ is selected from the group consisting of C₁-C₆-alkyl, C₂-C₆-alkenyl, C₂-C₆-alkynyl, C₁-C₆-alkoxy, C₂-C₆-alkenyloxy, C₂-C₆-alkynyloxy, where the 6 abovementioned radicals are unsubstituted or may be partially or fully halogenated and/or may carry 1, 2, 3, 4 or 5 substituents R^{ay}, where the substituents R^{ay} are independently of one another selected from the group consisting of cyano, nitro, hydroxyl, mercapto, amino, carboxyl, C₁-C₆-alkoxy, C₂-C₆-alkenyloxy, C₂-C₆-alkynyloxy, C₁-C₆-haloalkoxy, C₁-C₆-alkylthio, C₁-C₆-alkylamino, di(C₁-C₆-alkyl)amino, C₁-C₆-alkylsulfonyl, C₁-C₆-alkylsulfoxyl, formyl, C₁-C₆-alkylcarbonyl, C₁-C₆-alkoxycarbonyl, formyloxy and C₁-C₆-alkylcarbonyloxy;
C₃-C₁₄-cycloalkyl or phenyl which are unsubstituted or may be substituted by 1, 2, 3, 4 or 5 radicals R^{ax}, where the radicals R^{ax} are independently of one another selected from the group consisting of halogen, cyano, nitro, hydroxyl, mercapto, amino, carboxyl, C₁-C₆-alkyl, C₁-C₆-haloalkyl, C₃-C₆-cycloalkyl, C₁-C₆-alkoxy, C₂-C₆-alkenyloxy, C₂-C₆-alkynyloxy, C₁-C₆-haloalkoxy, C₁-C₆-alkylthio, C₁-C₆-alkylamino, di(C₁-C₆-alkyl)amino, C₁-C₆-alkylsulfonyl, C₁-C₆-alkylsulfoxyl, formyl, C₁-C₆-alkylcarbonyl, C₁-C₆-alkoxycarbonyl, formyloxy and C₁-C₆-alkylcarbonyloxy; comprising:
i) the provision of a pyrazolecarboxylate of the formula I by a process according to any of claims 1 to 10 and the reaction of the pyrazolecarboxylate of the formula I with an amino compound of the formula VI 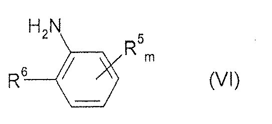 in which m, R⁵ and R⁶ have the meanings mentioned above;
or
ii) the provision of a pyrazole-4-carboxylic acid of the formula IV by a process according to claim 11, if appropriate the conversion of the pyrazole-4-carboxylic acid IV into its carbonyl halide and the subsequent reaction of the pyrazole-4-carboxylic acid of the formula IV or its carbonyl halide with an amino compound of the formula VI.

16. A compound of the formula A 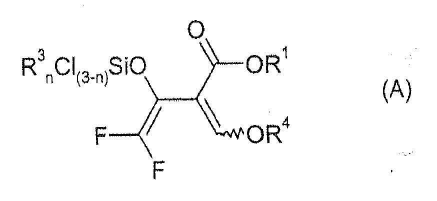 in which R¹ and R⁴ have one of the meanings given for formula II and R³ is independently selected from the group consisting of C₁-C₈-alkyl and phenyl.

## Revendications

1. Procédé pour la préparation de pyrazol-4-yl-carboxylates à substitution difluorométhyle, de formule générale (I), 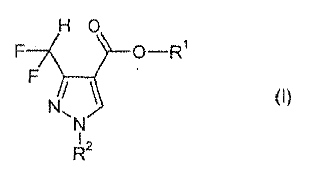 où
R¹ représente un groupe alkyle en C₁-C₈, cycloalkyle en C₃-C₈, alcoxy(C₁-C₄)-alkyle(C₁-C₄), cycloalcoxy(C₃-C₈)-alkyle(C₁-C₄), alcényle en C₂-C₈ ou un radical benzyle portant éventuellement 1, 2 ou 3 substituants R^{y1} choisis chacun indépendamment parmi des groupes alkyle en C₁-C₄, alcoxy en C₁-C₄ et nitro ; et
R² représente un atome d'hydrogène, un groupe alkyle en C₁-C₄, benzyle ou phényle, les deux substituants mentionnés en dernier pouvant être non substitués ou éventuellement porter 1, 2 ou 3 substituants R^{y2} choisis chacun indépendamment parmi des atomes d'halogène et des groupes CN, nitro, alkyle en C₁-C₄, halogénoalkyle en C₁-C₄, alcoxy en C₁-C₄ ou halogénoalcoxy en C₁-C₄ ;
dans lequel
a) on fait réagir un composé de formule générale (II) 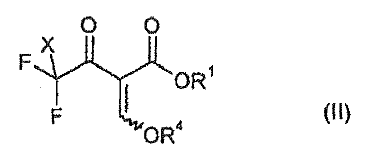 où X représente le fluor, le chlore ou le brome, R¹ a l'une des significations données précédemment et R⁴ représente un groupe alkyle en C₁-C₈, cycloalkyle en C₃-C₈, alcényle en C₂-C₈, benzyle ou phényle,
avec un composé silane de formule générale R³ₙSiCl₍₄₋ₙ₎,
où n représente 1, 2 ou 3 et les substituants R³ sont choisis chacun indépendamment parmi les groupes alkyle en C₁-C₈ ou phényle, et
avec un métal qui est choisi parmi les métaux des groupes 1, 2, 3, 4 et 12 du système périodique et présente un potentiel redox inférieur à -0,7 V, par rapport à une électrode à hydrogène normale (à 25 °C et sous 101,325 kPa) ; et
b) on fait réagir le mélange réactionnel provenant de l'étape a) avec un composé de formule générale (III)
R²HN-NH₂ (III)
où R² a l'une des significations données précédemment.

2. Procédé selon la revendication 1, dans lequel le métal est le magnésium.

3. Procédé selon l'une quelconque des revendications précédentes, dans lequel on utilise un composé silane de formule générale R³ₙSiCl₍₄₋ₙ₎, où n représente 2 ou 3.

4. Procédé selon l'une quelconque des revendications précédentes, dans lequel les substituants R³ dans le composé silane sont choisis chacun indépendamment parmi les groupes alkyle en C₁-C₄.

5. Procédé selon la revendication 3 ou 4, dans lequel le composé silane est choisi parmi le chlorure de triméthylsilyle, le chlorure d'éthyldiméthylsilyle, le chlorure de diméthylpropylsilyle, le chlorure de diméthylisopropylsilyle, le chlorure de n-butyldiméthylsilyle, le chlorure de 2-butyldiméthylsilyle, le chlorure de (2-méthylpropyl)diméthylsilyle, le diméthyldichlorosilane, le diéthyldichlorosilane et le chlorure de tert-butyldiméthylsilyle.

6. Procédé selon l'une quelconque des revendications précédentes, dans lequel R⁴ dans la formule II est choisi parmi un groupe alkyle en C₁-C₄ ou benzyle.

7. Procédé selon l'une quelconque des revendications précédentes, dans lequel la réaction dans l'étape a) s'effectue dans des conditions pratiquement anhydres.

8. Procédé selon la revendication 7, dans lequel la réaction dans l'étape a) s'effectue dans un solvant choisi parmi des N-alkyl(C₁-C₄)lactames, des N-di[alkyl(C₁-C₄)]amides d'acides carboxyliques aliphatiques en C₁-C₄, des N,N,N',N'-tétra[alkyl(C₁-C₄)]alkylurées, une 1,3-di[alkyl(C₁-C₄)]hexahydropyrimidin-2-one et une 1,3-di[alkyl(C₁-C₄)]imidazolin-2-one.

9. Procédé selon l'une quelconque des revendications précédentes, dans lequel on effectue la réaction dans l'étape b) en présence d'eau.

10. Procédé selon la revendication 9, dans lequel on effectue la réaction dans l'étape b) dans un mélange d'alcanol en C₁-C₄ et d'eau.

11. Procédé selon l'une quelconque des revendications précédentes, dans lequel R¹ dans les formules I et II représente un groupe alkyle en C₁-C₄ ou benzyle.

12. Procédé selon l'une quelconque des revendications précédentes, dans lequel le composé de formule générale (III) est choisi parmi les alkyl(C₁-C₄)-hydrazines et l'hydrate d'hydrazine.

13. Procédé pour la préparation d'un acide pyrazole-4-carboxylique de formule générale (IV) 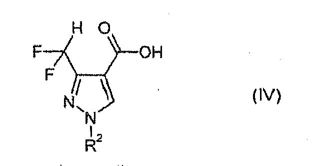 dans laquelle R² a l'une des significations données précédemment, comprenant
i) la préparation d'un composé de formule I conformément à un procédé selon l'une quelconque des revendications 1 à 10, et
ii) une hydrolyse du composé I en l'acide carboxylique IV.

14. Procédé selon la revendication 13, dans lequel l'hydrolyse basique s'effectue en présence d'une solution aqueuse d'hydroxyde de métal alcalin ou d'une solution aqueuse d'hydroxyde de métal alcalino-terreux.

15. Procédé pour la préparation d'anilides d'acide pyrazole-4-carboxylique de formule générale (V) 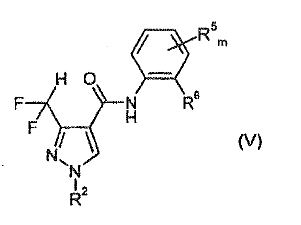 dans laquelle
R² a la signification donnée précédemment ;
R⁵ est choisi parmi un atome d'halogène, un groupe alkyle en C₁-C₆, alcoxy en C₁-C₆, alkyl(C₁-C₆)thio, halogénoalkyle en C₁-C₆, halogénoalcoxy en C₁-C₆ ou halogénoalkyl(C₁-C₆)thio ; m représente 0, 1, 2, 3 ou 4 ;
R⁶ est choisi parmi un groupe alkyle en C₁-C₆, alcényle en C₂-C₆, alcynyle en C₂-C₆, alcoxy en C₁-C₆, alcényl(C₂-C₆)oxy, alcynyl(C₂-C₆)oxy, les 6 radicaux précités étant non substitués ou pouvant être partiellement ou totalement halogénés et/ou pouvant porter 1, 2, 3, 4 ou 5 substituants R^{ay}, R^{ay} étant choisis, indépendamment les uns des autres, parmi les groupes cyano, nitro, hydroxy, mercapto, amino, carboxy, alcoxy en C₁-C₆, alcényl(C₂-C₆)oxy, alcynyl(C₂-C₆)oxy, halogénoalcoxy(C₁-C₆), alkyl(C₁-Cg)thio, alkyl(C₁-C₆)amino, di[alkyl(C₁-C₆)]amino, alkyl(C₁-C₆)sulfonyle, alkyl(C₁-C₆)sulfoxyle, formyle, alkyl(C₁-C₆)carbonyle, alcoxy(C₁-C₆)carbonyle, formyloxy et alkyl(C₂-C₆)-carbonyloxy ;
cycloalkyle en C₃-C₁₄ ou phényle, qui sont non substitués ou peuvent porter 1, 2, 3, 4 ou 5 substituants R^{ax}, R^{ax} étant choisis, indépendamment les uns des autres, parmi des atomes d'halogène et des groupes cyano, nitro, hydroxy, mercapto, amino, carboxy, alkyle en C₁-C₆, halogénoalkyle en C₁-C₆, cycloalkyle en C₃-C₆, alcoxy en C₁-C₆, alcényl(C₂-C₆)oxy, alcynyl(C₂-C₆)oxy, halogénoalcoxy(C₁-C₆), alkyl(C₁-C₆)thio, alkyl(C₁-C₆)amino, di[alkyl(C₁-C₆)]amino, alkyl(C₁-C₆)sulfonyle, alkyl(C₁-C₆)sulfoxyle, formyle, alkyl(C₁-C₆)carbonyle, alcoxy(C₁-C₆)carbonyle, formyloxy et alkyl(C₁-C₆)-carbonyloxy ;
comprenant
i) la préparation d'un pyrazole-carboxylate de formule I par un procédé selon l'une quelconque des revendications 1 à 10 et la réaction du pyrazole-carboxylate de formule I avec un composé amino de formule VI 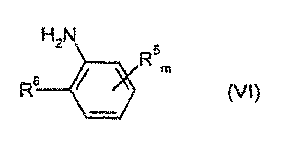 dans laquelle m, R⁵ et R⁶ ont les significations données précédemment ;
ou
ii) la préparation d'un acide pyrazole-4-carboxylique de formule IV par un procédé selon la revendication 11, éventuellement la conversion de l'acide pyrazole-4-carboxylique IV en son halogénure de carbonyle et la réaction subséquente de l'acide pyrazole-4-carboxylique de formule IV ou de son halogénure de carbonyle avec un composé amino de formule VI.

16. Composé de formule A 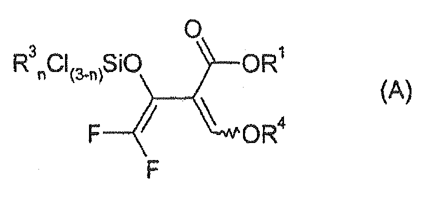 dans laquelle R¹ et R⁴ ont l'une des revendications indiquées pour la formule II et R³ sont choisis, indépendamment les uns des autres, parmi les groupes alkyle en C₁-C₈ ou phényle.
